# EUROPEAN PATENT SPECIFICATION

(11) **EP 2 823 053 B1**
(45) Date of publication and mention of the grant of the patent: **23.08.2017**
(21) Application number: 13707663.4
(22) Date of filing: 07.03.2013
(51) Int. Cl.: C12Q 1/37, C12Q 1/66, C07K 14/00, G01N 33/542

(54) **MEANS AND METHODS FOR DETERMINING NEUROTOXIN ACTIVITY BASED ON A MODIFIED LUCIFERASE**
MITTEL UND VERFAHREN ZUR BESTIMMUNG DER NEUROTOXINAKTIVITÄT BASIEREND AUF MODIFIZIERTER LUCIFERASE
MOYENS ET PROCÉDÉS POUR DÉTERMINER L'ACTIVITÉ DE NEUROTOXINES BASÉE SUR UNE LUCIFÉRASE MODIFIÉE

(30) Priority: 07.03.2012 EP 12158450; 07.03.2012 US 201261607760 P
(43) Date of publication of application: 14.01.2015
(73) Proprietor: Merz Pharma GmbH & Co. KGaA, 60318 Frankfurt am Main (DE)
(72) Inventor: EISELE, Karl-Heinz, 60385 Frankfurt am Main (DE)
(74) Representative: Herzog, Fiesser & Partner Patentanwälte PartG mbB
(86) International application number: PCT/EP2013/054566
(87) International publication number: WO 2013/131991

(56) References cited:
- WO-A2-2005/038029
- US-A1- 2009 176 259
- US-A1- 2010 297 620
- OLSSON O ET AL: "Engineering of monomeric bacterial luciferases by fusion of luxA and luxB genes in Vibrio harveyi", GENE, ELSEVIER, AMSTERDAM, NL, vol. 81, no. 2, 30 September 1989 (1989-09-30), pages 335-347, XP023544102, ISSN: 0378-1119, DOI: 10.1016/0378-1119(89)90194-7 [retrieved on 1989-09-30] cited in the application

### SEQUENCE LISTING

<110> Merz Pharma GmbH & Co.KGaA
<120> Means and methods for determining neurotoxin activity based on a modified luciferase
<130> MP10478PC
<150> EP 12 158 450.2
   <151> 2012-03-07
<150> US 61/607,760
   <151> 2012-03-07
<160> 14
<170> PatentIn version 3.5
<210> 1
   <211> 685
   <212> PRT
   <213> artificial
<220>
   <223> luciferase fusion protein LuxAB
<400> 1
   Met Lys Phe Gly Asn Phe Leu Leu Thr Tyr Gln Pro Pro Glu Leu Ser
   1               5                   10                  15
   Gln Thr Glu Val Met Lys Arg Leu Val Asn Leu Gly Lys Ala Ser Glu
               20                  25                  30
   Gly Cys Gly Phe Asp Thr Val Trp Leu Leu Glu His His Phe Thr Glu
           35                  40                  45
   Phe Gly Leu Leu Gly Asn Pro Tyr Val Ala Ala Ala His Leu Leu Gly
       50                  55                  60
   Ala Thr Glu Thr Leu Asn Val Gly Thr Ala Ala Ile Val Leu Pro Thr
   65                  70                  75                  80
   Ala His Pro Val Arg Gln Ala Glu Asp Val Asn Leu Leu Asp Gln Met
                   85                  90                  95
   Ser Lys Gly Arg Phe Arg Phe Gly Ile Cys Arg Gly Leu Tyr Asp Lys
               100                 105                 110
   Asp Phe Arg Val Phe Gly Thr Asp Met Asp Asn Ser Arg Ala Leu Met
           115                 120                 125
   Asp Cys Trp Tyr Asp Leu Met Lys Glu Gly Phe Asn Glu Gly Tyr Ile
       130                 135                 140
   Ala Ala Asp Asn Glu His Ile Lys Phe Pro Lys Ile Gln Leu Asn Pro
   145                 150                 155                 160
   Ser Ala Tyr Thr Gln Gly Gly Ala Pro Val Tyr Val Val Ala Glu Ser
                   165                 170                 175
   Ala Ser Thr Thr Glu Trp Ala Ala Glu Arg Gly Leu Pro Met Ile Leu
               180                 185                 190
   Ser Trp Ile Ile Asn Thr His Glu Lys Lys Ala Gln Leu Asp Leu Tyr
           195                 200                 205
   Asn Glu Val Ala Thr Glu His Gly Tyr Asp Val Thr Lys Ile Asp His
       210                 215                 220
   Cys Leu Ser Tyr Ile Thr Ser Val Asp His Asp Ser Asn Arg Ala Lys
   225                 230                 235                 240
   Asp Ile Cys Arg Asn Phe Leu Gly His Trp Tyr Asp Ser Tyr Val Asn
                   245                 250                 255
   Ala Thr Lys Ile Phe Asp Asp Ser Asp Gln Thr Lys Gly Tyr Asp Phe
               260                 265                 270
   Asn Lys Gly Gln Trp Arg Asp Phe Val Leu Lys Gly His Lys Asp Thr
           275                 280                 285
   Asn Arg Arg Ile Asp Tyr Ser Tyr Glu Ile Asn Pro Val Gly Thr Pro
       290                 295                 300
   Glu Glu Cys Ile Ala Ile Ile Gln Gln Asp Ile Asp Ala Thr Gly Ile
   305                 310                 315                 320
   Asp Asn Ile Cys Cys Gly Phe Glu Ala Asn Gly Ser Glu Glu Glu Ile
                   325                 330                 335
   Ile Ala Ser Met Lys Leu Phe Gln Ser Asp Val Met Pro Tyr Leu Lys
               340                 345                 350
   Glu Lys Gln Tyr Leu Ile Phe Ser Gln Lys Glu Arg Asp Lys Lys Phe
           355                 360                 365
   Gly Leu Phe Phe Leu Asn Phe Met Asn Ser Lys Arg Ser Ser Asp Gln
       370                 375                 380
   Val Ile Glu Glu Met Leu Asp Thr Ala His Tyr Val Asp Gln Leu Lys
   385                 390                 395                 400
   Phe Asp Thr Leu Ala Val Tyr Glu Asn His Phe Ser Asn Asn Gly Val
                   405                 410                 415
   Val Gly Ala Pro Leu Thr Val Ala Gly Phe Leu Leu Gly Met Thr Lys
               420                 425                 430
   Asn Ala Lys Val Ala Ser Leu Asn His Val Ile Thr Thr His His Pro
           435                 440                 445
   Val Arg Val Ala Glu Glu Ala Cys Leu Leu Asp Gln Met Ser Glu Gly
       450                 455                 460
   Arg Phe Ala Phe Gly Phe Ser Asp Cys Glu Lys Ser Ala Asp Met Arg
   465                 470                 475                 480
   Phe Phe Asn Arg Pro Thr Asp Ser Gln Phe Gln Leu Phe Ser Glu Cys
                   485                 490                 495
   His Lys Ile Ile Asn Asp Ala Phe Thr Thr Gly Tyr Cys His Pro Asn
               500                 505                 510
   Asn Asp Phe Tyr Ser Phe Pro Lys Ile Ser Val Asn Pro His Ala Phe
           515                 520                 525
   Thr Glu Gly Gly Pro Ala Gln Phe Val Asn Ala Thr Ser Lys Glu Val
       530                 535                 540
   Val Glu Trp Ala Ala Lys Leu Gly Leu Pro Leu Val Phe Arg Trp Asp
   545                 550                 555                 560
   Asp Ser Asn Ala Gln Arg Lys Glu Tyr Ala Gly Leu Tyr His Glu Val
                   565                 570                 575
   Ala Gln Ala His Gly Val Asp Val Ser Gln Val Arg His Lys Leu Thr
               580                 585                 590
   Leu Leu Val Asn Gln Asn Val Asp Gly Glu Ala Ala Arg Ala Glu Ala
           595                 600                 605
   Arg Val Tyr Leu Glu Glu Phe Val Arg Glu Ser Tyr Ser Asn Thr Asp
       610                 615                 620
   Phe Glu Gln Lys Met Gly Glu Leu Leu Ser Glu Asn Ala Ile Gly Thr
   625                 630                 635                 640
   Tyr Glu Glu Ser Thr Gln Ala Ala Arg Val Ala Ile Glu Cys Cys Gly
                   645                 650                 655
   Ala Ala Asp Leu Leu Met Ser Phe Glu Ser Met Glu Asp Lys Ala Gln
               660                 665                 670
   Gln Arg Ala Val Ile Asp Val Val Asn Ala Asn Ile Val
           675                 680                 685
<210> 2
   <211> 691
   <212> PRT
   <213> artificial
<220>
   <223> luciferase fusion protein LuxBA_1
<400> 2
   Met Lys Phe Gly Leu Phe Phe Leu Asn Phe Met Asn Ser Lys Arg Ser
   1 5 10 15
   Ser Asp Gln Val Ile Glu Glu Met Leu Asp Thr Ala His Tyr Val Asp
   20 25 30
   Gln Leu Lys Phe Asp Thr Leu Ala Val Tyr Glu Asn His Phe Ser Asn
   35 40 45
   Asn Gly Val Val Gly Ala Pro Leu Thr Val Ala Gly Phe Leu Leu Gly
   50 55 60
   Met Thr Lys Asn Ala Lys Val Ala Ser Leu Asn His Val Ile Thr Thr
   65 70 75 80
   His His Pro Val Arg Val Ala Glu Glu Ala Cys Leu Leu Asp Gln Met
   85 90 95
   Ser Glu Gly Arg Phe Ala Phe Gly Phe Ser Asp Cys Glu Lys Ser Ala
   100 105 110
   Asp Met Arg Phe Phe Asn Arg Pro Thr Asp Ser Gln Phe Gln Leu Phe
   115 120 125
   Ser Glu Cys His Lys Ile Ile Asn Asp Ala Phe Thr Thr Gly Tyr Cys
   130 135 140
   His Pro Asn Asn Asp Phe Tyr Ser Phe Pro Lys Ile Ser Val Asn Pro
   145 150 155 160
   His Ala Phe Thr Glu Gly Gly Pro Ala Gln Phe Val Asn Ala Thr Ser
                   165                 170                 175
   Lys Glu Val Val Glu Trp Ala Ala Lys Leu Gly Leu Pro Leu Val Phe
               180                 185                 190
   Arg Trp Asp Asp Ser Asn Ala Gln Arg Lys Glu Tyr Ala Gly Leu Tyr
           195                 200                 205
   His Glu Val Ala Gln Ala His Gly Val Asp Val Ser Gln Val Arg His
       210                 215                 220
   Lys Leu Thr Leu Leu Val Asn Gln Asn Val Asp Gly Glu Ala Ala Arg
   225                 230                 235                 240
   Ala Glu Ala Arg Val Tyr Leu Glu Glu Phe Val Arg Glu Ser Tyr Ser
                   245                 250                 255
   Asn Thr Asp Phe Glu Gln Lys Met Gly Glu Leu Leu Ser Glu Asn Ala
               260                 265                 270
   Ile Gly Thr Tyr Glu Glu Ser Thr Gln Ala Ala Arg Val Ala Ile Glu
           275                 280                 285
   Cys Cys Gly Ala Ala Asp Leu Leu Met Ser Phe Glu Ser Met Glu Asp
       290                 295                 300
   Lys Ala Gln Gln Arg Ala Val Ile Asp Val Val Ala Asn Ile Val Thr
   305                 310                 315                 320
   Arg Ile Asp Glu Ala Asn Gln Arg Ala Thr Lys Met Leu Gly Ser Gly
                   325                 330                 335
   Met Lys Phe Gly Asn Phe Leu Leu Thr Tyr Gln Pro Pro Glu Leu Ser
               340                 345                 350
   Gln Thr Glu Val Met Lys Arg Leu Val Asn Leu Gly Lys Ala Ser Glu
           355                 360                 365
   Gly Cys Gly Phe Asp Thr Val Trp Leu Leu Glu His His Phe Thr Glu
       370                 375                 380
   Phe Gly Leu Leu Gly Asn Pro Tyr Val Ala Ala Ala His Leu Leu Gly
   385                 390                 395                 400
   Ala Thr Glu Thr Leu Asn Val Gly Thr Ala Ala Ile Val Leu Pro Thr
                   405                 410                 415
   Ala His Pro Val Arg Gln Ala Glu Asp Val Asn Leu Leu Asp Gln Met
               420                 425                 430
   Ser Lys Gly Arg Phe Arg Phe Gly Ile Cys Arg Gly Leu Tyr Asp Lys
           435                 440                 445
   Asp Phe Arg Val Phe Gly Thr Asp Met Asp Asn Ser Arg Ala Leu Met
       450                 455                 460
   Asp Cys Trp Tyr Asp Leu Met Lys Glu Gly Phe Asn Glu Gly Tyr Ile
   465                 470                 475                 480
   Ala Ala Asp Asn Glu His Ile Lys Phe Pro Lys Ile Gln Leu Asn Pro
                   485                 490                 495
   Ser Ala Tyr Thr Gln Gly Gly Ala Pro Val Tyr Val Val Ala Glu Ser
               500                 505                 510
   Ala Ser Thr Thr Glu Trp Ala Ala Glu Arg Gly Leu Pro Met Ile Leu
           515                 520                 525
   Ser Trp Ile Ile Asn Thr His Glu Lys Lys Ala Gln Leu Asp Leu Tyr
       530                 535                 540
   Asn Glu Val Ala Thr Glu His Gly Tyr Asp Val Thr Lys Ile Asp His
   545                 550                 555                 560
   Cys Leu Ser Tyr Ile Thr Ser Val Asp His Asp Ser Asn Arg Ala Lys
                   565                 570                 575
   Asp Ile Cys Arg Asn Phe Leu Gly His Trp Tyr Asp Ser Tyr Val Asn
               580                 585                 590
   Ala Thr Lys Ile Phe Asp Asp Ser Asp Gln Thr Lys Gly Tyr Asp Phe
           595                 600                 605
   Asn Lys Gly Gln Trp Arg Asp Phe Val Leu Lys Gly His Lys Asp Thr
       610                 615                 620
   Asn Arg Arg Ile Asp Tyr Ser Tyr Glu Ile Asn Pro Val Gly Thr Pro
   625                 630                 635                 640
   Glu Glu Cys Ile Ala Ile Ile Gln Gln Asp Ile Asp Ala Thr Gly Ile
                   645                 650                 655
   Asp Asn Ile Cys Cys Gly Phe Glu Ala Asn Gly Ser Glu Glu Glu Ile
               660                 665                 670
   Ile Ala Ser Met Lys Leu Phe Gln Ser Asp Val Met Pro Tyr Leu Lys
   675 680 685
   Glu Lys Gln
   690
<210> 3
   <211> 741
   <212> PRT
   <213> artificial
<220>
   <223> luciferase fusion protein LuxBA_3
<400> 3
   Met Lys Phe Gly Leu Phe Phe Leu Asn Phe Met Asn Ser Lys Arg Ser
   1 5 10 15
   Ser Asp Gln Val Ile Glu Glu Met Leu Asp Thr Ala His Tyr Val Asp
   20 25 30
   Gln Leu Lys Phe Asp Thr Leu Ala Val Tyr Glu Asn His Phe Ser Asn
   35 40 45
   Asn Gly Val Val Gly Ala Pro Leu Thr Val Ala Gly Phe Leu Leu Gly
   50 55 60
   Met Thr Lys Asn Ala Lys Val Ala Ser Leu Asn His Val Ile Thr Thr
   65 70 75 80
   His His Pro Val Arg Val Ala Glu Glu Ala Cys Leu Leu Asp Gln Met
   85 90 95
   Ser Glu Gly Arg Phe Ala Phe Gly Phe Ser Asp Cys Glu Lys Ser Ala
   100 105 110
   Asp Met Arg Phe Phe Asn Arg Pro Thr Asp Ser Gln Phe Gln Leu Phe
   115 120 125
   Ser Glu Cys His Lys Ile Ile Asn Asp Ala Phe Thr Thr Gly Tyr Cys
   130 135 140
   His Pro Asn Asn Asp Phe Tyr Ser Phe Pro Lys Ile Ser Val Asn Pro
   145 150 155 160
   His Ala Phe Thr Glu Gly Gly Pro Ala Gln Phe Val Asn Ala Thr Ser
   165 170 175
   Lys Glu Val Val Glu Trp Ala Ala Lys Leu Gly Leu Pro Leu Val Phe
               180                 185                 190
   Arg Trp Asp Asp Ser Asn Ala Gln Arg Lys Glu Tyr Ala Gly Leu Tyr
           195                 200                 205
   His Glu Val Ala Gln Ala His Gly Val Asp Val Ser Gln Val Arg His
       210                 215                 220
   Lys Leu Thr Leu Leu Val Asn Gln Asn Val Asp Gly Glu Ala Ala Arg
   225                 230                 235                 240
   Ala Glu Ala Arg Val Tyr Leu Glu Glu Phe Val Arg Glu Ser Tyr Ser
                   245                 250                 255
   Asn Thr Asp Phe Glu Gln Lys Met Gly Glu Leu Leu Ser Glu Asn Ala
               260                 265                 270
   Ile Gly Thr Tyr Glu Glu Ser Thr Gln Ala Ala Arg Val Ala Ile Glu
           275                 280                 285
   Cys Cys Gly Ala Ala Asp Leu Leu Met Ser Phe Glu Ser Met Glu Asp
       290                 295                 300
   Lys Ala Gln Gln Arg Ala Val Ile Asp Val Val Arg Arg Val Thr Asp
   305                 310                 315                 320
   Ala Arg Glu Asn Glu Met Asp Glu Asn Leu Glu Gln Val Ser Gly Ile
                   325                 330                 335
   Ile Gly Asn Leu Arg His Met Ala Leu Asp Met Gly Asn Glu Ile Asp
               340                 345                 350
   Thr Gln Asn Arg Gln Ile Asp Arg Ile Met Glu Lys Ala Asp Ser Asn
           355                 360                 365
   Lys Thr Arg Ile Asp Glu Ala Asn Gln Arg Ala Thr Lys Met Leu Gly
       370                 375                 380
   Ser Gly Lys Lys Phe Gly Asn Phe Leu Leu Thr Tyr Gln Pro Pro Glu
   385                 390                 395                 400
   Leu Ser Gln Thr Glu Val Met Lys Arg Leu Val Asn Leu Gly Lys Ala
                   405                 410                 415
   Ser Glu Gly Cys Gly Phe Asp Thr Val Trp Leu Leu Glu His His Phe
               420                 425                 430
   Thr Glu Phe Gly Leu Leu Gly Asn Pro Tyr Val Ala Ala Ala His Leu
   435 440 445
   Leu Gly Ala Thr Glu Thr Leu Asn Val Gly Thr Ala Ala Ile Val Leu
   450 455 460
   Pro Thr Ala His Pro Val Arg Gln Ala Glu Asp Val Asn Leu Leu Asp
   465 470 475 480
   Gln Met Ser Lys Gly Arg Phe Arg Phe Gly Ile Cys Arg Gly Leu Tyr
   485 490 495
   Asp Lys Asp Phe Arg Val Phe Gly Thr Asp Met Asp Asn Ser Arg Ala
   500 505 510
   Leu Met Asp Cys Trp Tyr Asp Leu Met Lys Glu Gly Phe Asn Glu Gly
   515 520 525
   Tyr Ile Ala Ala Asp Asn Glu His Ile Lys Phe Pro Lys Ile Gln Leu
   530 535 540
   Asn Pro Ser Ala Tyr Thr Gln Gly Gly Ala Pro Val Tyr Val Val Ala
   545 550 555 560
   Glu Ser Ala Ser Thr Thr Glu Trp Ala Ala Glu Arg Gly Leu Pro Met
   565 570 575
   Ile Leu Ser Trp Ile Ile Asn Thr His Glu Lys Lys Ala Gln Leu Asp
   580 585 590
   Leu Tyr Asn Glu Val Ala Thr Glu His Gly Tyr Asp Val Thr Lys Ile
   595 600 605
   Asp His Cys Leu Ser Tyr Ile Thr Ser Val Asp His Asp Ser Asn Arg
   610 615 620
   Ala Lys Asp Ile Cys Arg Asn Phe Leu Gly His Trp Tyr Asp Ser Tyr
   625 630 635 640
   Val Asn Ala Thr Lys Ile Phe Asp Asp Ser Asp Gln Thr Lys Gly Tyr
   645 650 655
   Asp Phe Asn Lys Gly Gln Trp Arg Asp Phe Val Leu Lys Gly His Lys
   660 665 670
   Asp Thr Asn Arg Arg Ile Asp Tyr Ser Tyr Glu Ile Asn Pro Val Gly
   675 680 685
   Thr Pro Glu Glu Cys Ile Ala Ile Ile Gln Gln Asp Ile Asp Ala Thr
   690 695 700
   Gly Ile Asp Asn Ile Cys Cys Gly Phe Glu Ala Asn Gly Ser Glu Glu
   705 710 715 720
   Glu Ile Ile Ala Ser Met Lys Leu Phe Gln Ser Asp Val Met Pro Tyr
   725 730 735
   Leu Lys Glu Lys Gln
   740
<210> 4
   <211> 319
   <212> PRT
   <213> Vibrio fischeri
<400> 4
   Lys Phe Gly Leu Phe Phe Leu Asn Phe Met Asn Ser Lys Arg Ser Ser
   1 5 10 15
   Asp Gln Val Ile Glu Glu Met Leu Asp Thr Ala His Tyr Val Asp Gln
   20 25 30
   Leu Lys Phe Asp Thr Leu Ala Val Tyr Glu Asn His Phe Ser Asn Asn
   35 40 45
   Gly Val Val Gly Ala Pro Leu Thr Val Ala Gly Phe Leu Leu Gly Met
   50 55 60
   Thr Lys Asn Ala Lys Val Ala Ser Leu Asn His Val Ile Thr Thr His
   65 70 75 80
   His Pro Val Arg Val Ala Glu Glu Ala Cys Leu Leu Asp Gln Met Ser
   85 90 95
   Glu Gly Arg Phe Ala Phe Gly Phe Ser Asp Cys Glu Lys Ser Ala Asp
   100 105 110
   Met Arg Phe Phe Asn Arg Pro Thr Asp Ser Gln Phe Gln Leu Phe Ser
   115 120 125
   Glu Cys His Lys Ile Ile Asn Asp Ala Phe Thr Thr Gly Tyr Cys His
   130 135 140
   Pro Asn Asn Asp Phe Tyr Ser Phe Pro Lys Ile Ser Val Asn Pro His
   145 150 155 160
   Ala Phe Thr Glu Gly Gly Pro Ala Gln Phe Val Asn Ala Thr Ser Lys
   165 170 175
   Glu Val Val Glu Trp Ala Ala Lys Leu Gly Leu Pro Leu Val Phe Arg
   180 185 190
   Trp Asp Asp Ser Asn Ala Gln Arg Lys Glu Tyr Ala Gly Leu Tyr His
   195 200 205
   Glu Val Ala Gln Ala His Gly Val Asp Val Ser Gln Val Arg His Lys
   210 215 220
   Leu Thr Leu Leu Val Asn Gln Asn Val Asp Gly Glu Ala Ala Arg Ala
   225 230 235 240
   Glu Ala Arg Val Tyr Leu Glu Glu Phe Val Arg Glu Ser Tyr Ser Asn
   245 250 255
   Thr Asp Phe Glu Gln Lys Met Gly Glu Leu Leu Ser Glu Asn Ala Ile
   260 265 270
   Gly Thr Tyr Glu Glu Ser Thr Gln Ala Ala Arg Val Ala Ile Glu Cys
   275 280 285
   Cys Gly Ala Ala Asp Leu Leu Met Ser Phe Glu Ser Met Glu Asp Lys
   290 295 300
   Ala Gln Gln Arg Ala Val Ile Asp Val Val Asn Ala Asn Ile Val
   305 310 315
<210> 5
   <211> 355
   <212> PRT
   <213> Vibrio fischeri
<400> 5
   Met Lys Phe Gly Asn Phe Leu Leu Thr Tyr Gln Pro Pro Glu Leu Ser
   1 5 10 15
   Gln Thr Glu Val Met Lys Arg Leu Val Asn Leu Gly Lys Ala Ser Glu
   20 25 30
   Gly Cys Gly Phe Asp Thr Val Trp Leu Leu Glu His His Phe Thr Glu
   35 40 45
   Phe Gly Leu Leu Gly Asn Pro Tyr Val Ala Ala Ala His Leu Leu Gly
   50 55 60
   Ala Thr Glu Thr Leu Asn Val Gly Thr Ala Ala Ile Val Leu Pro Thr
   65 70 75 80
   Ala His Pro Val Arg Gln Ala Glu Asp Val Asn Leu Leu Asp Gln Met
   85 90 95
   Ser Lys Gly Arg Phe Arg Phe Gly Ile Cys Arg Gly Leu Tyr Asp Lys
   100 105 110
   Asp Phe Arg Val Phe Gly Thr Asp Met Asp Asn Ser Arg Ala Leu Met
   115 120 125
   Asp Cys Trp Tyr Asp Leu Met Lys Glu Gly Phe Asn Glu Gly Tyr Ile
   130 135 140
   Ala Ala Asp Asn Glu His Ile Lys Phe Pro Lys Ile Gln Leu Asn Pro
   145 150 155 160
   Ser Ala Tyr Thr Gln Gly Gly Ala Pro Val Tyr Val Val Ala Glu Ser
   165 170 175
   Ala Ser Thr Thr Glu Trp Ala Ala Glu Arg Gly Leu Pro Met Ile Leu
   180 185 190
   Ser Trp Ile Ile Asn Thr His Glu Lys Lys Ala Gln Leu Asp Leu Tyr
   195 200 205
   Asn Glu Val Ala Thr Glu His Gly Tyr Asp Val Thr Lys Ile Asp His
   210 215 220
   Cys Leu Ser Tyr Ile Thr Ser Val Asp His Asp Ser Asn Arg Ala Lys
   225 230 235 240
   Asp Ile Cys Arg Asn Phe Leu Gly His Trp Tyr Asp Ser Tyr Val Asn
   245 250 255
   Ala Thr Lys Ile Phe Asp Asp Ser Asp Gln Thr Lys Gly Tyr Asp Phe
   260 265 270
   Asn Lys Gly Gln Trp Arg Asp Phe Val Leu Lys Gly His Lys Asp Thr
   275 280 285
   Asn Arg Arg Ile Asp Tyr Ser Tyr Glu Ile Asn Pro Val Gly Thr Pro
   290 295 300
   Glu Glu Cys Ile Ala Ile Ile Gln Gln Asp Ile Asp Ala Thr Gly Ile
   305 310 315 320
   Asp Asn Ile Cys Cys Gly Phe Glu Ala Asn Gly Ser Glu Glu Glu Ile
   325 330 335
   Ile Ala Ser Met Lys Leu Phe Gln Ser Asp Val Met Pro Tyr Leu Lys
   340 345 350
   Glu Lys Gln
   355
<210> 6
   <211> 17
   <212> PRT
   <213> artificial
<220>
   <223> SNAP25 linker 1
<400> 6
   Thr Arg Ile Asp Glu Ala Asn Gln Arg Ala Thr Lys Met Leu Gly Ser
   1 5 10 15
   Gly
<210> 7
   <211> 13
   <212> PRT
   <213> artificial
<220>
   <223> SNAP25 linker 2
<400> 7
   Ile Asp Glu Ala Asn Gln Arg Ala Thr Lys Met Leu Gly
   1 5 10
<210> 8
   <211> 72
   <212> PRT
   <213> artificial
<220>
   <223> SNAP25 linker 3
<400> 8
   Arg Arg Val Thr Asp Ala Arg Glu Asn Glu Met Asp Glu Asn Leu Glu
   1               5                   10                  15
   Gln Val Ser Gly Ile Ile Gly Asn Leu Arg His Met Ala Leu Asp Met
               20                  25                  30
   Gly Asn Glu Ile Asp Thr Gln Asn Arg Gln Ile Asp Arg Ile Met Glu
           35                  40                  45
   Lys Ala Asp Ser Asn Lys Thr Arg Ile Asp Glu Ala Asn Gln Arg Ala
       50                  55                  60
   Thr Lys Met Leu Gly Ser Gly Lys
   65                  70
<210> 9
   <211> 5230
   <212> DNA
   <213> Artificial Sequence
<220>
   <223> construct MRZ_LuxAB0; DNA vector comprising LuxAB0 sequence
<400> 9
   ccatcgaatg gccagatgat taattcctaa tttttgttga cactctatca ttgatagagt      60
   tattttacca ctccctatca gtgatagaga aaagtgaaat gaatagttcg acaaaaatct     120
   agaaataatt ttgtttaact ttaagaagga gatatacaaa tgaagttcgg caacttcctg     180
   ctgacctacc agccccctga gctgagccag accgaagtga tgaagaggct ggtgaacctg     240
   ggcaaggcca gcgagggctg tggcttcgac accgtgtggc tgctggaaca ccacttcacc     300
   gagttcggcc tgctgggcaa cccttacgtg gccgctgccc atctgctggg cgccaccgag     360
   acactgaacg tgggcaccgc cgccattgtg ctgcctacag cccaccctgt gcggcaggcc     420
   gaggacgtga acctgctgga ccagatgagc aagggcaggt tcagattcgg catctgcagg     480
   ggcctgtacg acaaggactt cagggtgttc ggcaccgaca tggacaacag cagggctctg     540
   atggactgtt ggtacgacct gatgaaggaa ggcttcaacg agggctacat tgccgccgac     600
   aacgagcaca tcaagttccc caagatccag ctgaacccca gcgcctacac acagggcgga     660
   gcccctgtgt acgtggtggc cgagagcgcc tctacaaccg agtgggccgc tgagaggggc     720
   ctgcccatga tcctgagctg gatcatcaac acccacgaga agaaggccca gctggacctg     780
   tacaacgagg tggccacaga gcacggctac gacgtgacca agatcgacca ctgcctgagc     840
   tacatcacca gcgtggacca cgacagcaac agggccaagg acatctgcag gaactttctg     900
   ggccattggt acgacagcta cgtgaacgcc accaagatct tcgacgacag cgaccagacc     960
   aagggctacg acttcaacaa gggccagtgg agggacttcg tgctgaaggg ccacaaggac    1020
   accaacaggc ggatcgacta cagctacgag atcaaccccg tgggcacccc cgaggaatgt    1080
   atcgccatca tccagcagga catcgacgcc accggcatcg acaacatctg ctgcggcttc    1140
   gaggccaacg gcagcgagga agagatcatt gccagcatga agctgttcca gagcgacgtg    1200
   atgccctacc tgaaagagaa gcagtacctg atcttcagcc agaaagagag ggacaagaag 1260
   ttcgggctgt tcttcctgaa cttcatgaac agcaagaggt ccagcgacca ggtgatcgag 1320
   gaaatgctgg acaccgccca ctacgtggac cagctgaagt tcgacaccct ggccgtgtac 1380
   gagaaccact tcagcaacaa cggcgtggtg ggagcccctc tgacagtggc cggcttcctg 1440
   ctgggaatga ccaagaacgc caaggtggcc agcctgaacc acgtgatcac cacccaccat 1500
   cctgtgcggg tggccgaaga ggcctgtctg ctggatcaga tgtccgaggg cagattcgcc 1560
   ttcggcttca gcgactgcga gaagtccgcc gacatgcggt tcttcaacag gcccaccgac 1620
   agccagttcc agctgttcag cgagtgccac aagatcatca acgacgcctt caccaccggc 1680
   tactgccacc ccaacaacga cttctacagc ttccctaaga tctccgtgaa cccccacgcc 1740
   tttacagagg gcggacccgc ccagttcgtg aacgctacca gcaaggaagt ggtggagtgg 1800
   gctgccaagc tgggcctgcc cctggtgttc agatgggacg actccaacgc ccagaggaaa 1860
   gagtacgccg gcctgtacca tgaagtggct caggctcacg gcgtggacgt gtcccaggtc 1920
   cggcacaagc tgaccctgct ggtgaaccag aacgtggacg gcgaggccgc tagagccgag 1980
   gccagggtgt acctggaaga gttcgtgcgg gagagctaca gcaacaccga cttcgagcag 2040
   aagatgggcg agctgctgag cgagaacgcc atcggcacct acgaggaaag cacccaggcc 2100
   gccagagtgg ccatcgagtg ctgtggagcc gccgacctgc tgatgagctt cgagagcatg 2160
   gaagataagg cccagcagag ggccgtgatc gacgtggtga acgccaacat cgtgggtctc 2220
   agcgcttgga gccacccgca gttcgaaaaa taataagctt gacctgtgaa gtgaaaaatg 2280
   gcgcacattg tgcgacattt tttttgtctg ccgtttaccg ctactgcgtc acggatctcc 2340
   acgcgccctg tagcggcgca ttaagcgcgg cgggtgtggt ggttacgcgc agcgtgaccg 2400
   ctacacttgc cagcgcccta gcgcccgctc ctttcgcttt cttcccttcc tttctcgcca 2460
   cgttcgccgg ctttccccgt caagctctaa atcgggggct ccctttaggg ttccgattta 2520
   gtgctttacg gcacctcgac cccaaaaaac ttgattaggg tgatggttca cgtagtgggc 2580
   catcgccctg atagacggtt tttcgccctt tgacgttgga gtccacgttc tttaatagtg 2640
   gactcttgtt ccaaactgga acaacactca accctatctc ggtctattct tttgatttat 2700
   aagggatttt gccgatttcg gcctattggt taaaaaatga gctgatttaa caaaaattta 2760
   acgcgaattt taacaaaata ttaacgctta caatttcagg tggcactttt cggggaaatg 2820
   tgcgcggaac ccctatttgt ttatttttct aaatacattc aaatatgtat ccgctcatga 2880
   gacaataacc ctgataaatg cttcaataat attgaaaaag gaagagtatg agtattcaac 2940
   atttccgtgt cgcccttatt cccttttttg cggcattttg ccttcctgtt tttgctcacc 3000
   cagaaacgct ggtgaaagta aaagatgctg aagatcagtt gggtgcacga gtgggttaca 3060
   tcgaactgga tctcaacagc ggtaagatcc ttgagagttt tcgccccgaa gaacgttttc 3120
   caatgatgag cacttttaaa gttctgctat gtggcgcggt attatcccgt attgacgccg 3180
   ggcaagagca actcggtcgc cgcatacact attctcagaa tgacttggtt gagtactcac 3240
   cagtcacaga aaagcatctt acggatggca tgacagtaag agaattatgc agtgctgcca 3300
   taaccatgag tgataacact gcggccaact tacttctgac aacgatcgga ggaccgaagg 3360
   agctaaccgc ttttttgcac aacatggggg atcatgtaac tcgccttgat cgttgggaac 3420
   cggagctgaa tgaagccata ccaaacgacg agcgtgacac cacgatgcct gtagcaatgg 3480
   caacaacgtt gcgcaaacta ttaactggcg aactacttac tctagcttcc cggcaacaat 3540
   tgatagactg gatggaggcg gataaagttg caggaccact tctgcgctcg gcccttccgg 3600
   ctggctggtt tattgctgat aaatctggag ccggtgagcg tggctctcgc ggtatcattg 3660
   cagcactggg gccagatggt aagccctccc gtatcgtagt tatctacacg acggggagtc 3720
   aggcaactat ggatgaacga aatagacaga tcgctgagat aggtgcctca ctgattaagc 3780
   attggtagga attaatgatg tctcgtttag ataaaagtaa agtgattaac agcgcattag 3840
   agctgcttaa tgaggtcgga atcgaaggtt taacaacccg taaactcgcc cagaagctag 3900
   gtgtagagca gcctacattg tattggcatg taaaaaataa gcgggctttg ctcgacgcct 3960
   tagccattga gatgttagat aggcaccata ctcacttttg ccctttagaa ggggaaagct 4020
   ggcaagattt tttacgtaat aacgctaaaa gttttagatg tgctttacta agtcatcgcg 4080
   atggagcaaa agtacattta ggtacacggc ctacagaaaa acagtatgaa actctcgaaa 4140
   atcaattagc ctttttatgc caacaaggtt tttcactaga gaatgcatta tatgcactca 4200
   gcgcagtggg gcattttact ttaggttgcg tattggaaga tcaagagcat caagtcgcta 4260
   aagaagaaag ggaaacacct actactgata gtatgccgcc attattacga caagctatcg 4320
   aattatttga tcaccaaggt gcagagccag ccttcttatt cggccttgaa ttgatcatat 4380
   gcggattaga aaaacaactt aaatgtgaaa gtgggtctta aaagcagcat aacctttttc 4440
   cgtgatggta acttcactag tttaaaagga tctaggtgaa gatccttttt gataatctca 4500
   tgaccaaaat cccttaacgt gagttttcgt tccactgagc gtcagacccc gtagaaaaga 4560
   tcaaaggatc ttcttgagat cctttttttc tgcgcgtaat ctgctgcttg caaacaaaaa 4620
   aaccaccgct accagcggtg gtttgtttgc cggatcaaga gctaccaact ctttttccga 4680
   aggtaactgg cttcagcaga gcgcagatac caaatactgt ccttctagtg tagccgtagt 4740
   taggccacca cttcaagaac tctgtagcac cgcctacata cctcgctctg ctaatcctgt 4800
   taccagtggc tgctgccagt ggcgataagt cgtgtcttac cgggttggac tcaagacgat 4860
   agttaccgga taaggcgcag cggtcgggct gaacgggggg ttcgtgcaca cagcccagct 4920
   tggagcgaac gacctacacc gaactgagat acctacagcg tgagctatga gaaagcgcca 4980
   cgcttcccga agggagaaag gcggacaggt atccggtaag cggcagggtc ggaacaggag 5040
   agcgcacgag ggagcttcca gggggaaacg cctggtatct ttatagtcct gtcgggtttc 5100
   gccacctctg acttgagcgt cgatttttgt gatgctcgtc aggggggcgg agcctatgga 5160
   aaaacgccag caacgcggcc tttttacggt tcctggcctt ttgctggcct tttgctcaca 5220
   tgacccgaca 5230
<210> 10
   <211> 5398
   <212> DNA
   <213> artificial sequences
<220>
   <223> construct MRZ_LuxBA3; DNA vector comprising LuxBA3 sequence
<400> 10
   ccatcgaatg gccagatgat taattcctaa tttttgttga cactctatca ttgatagagt      60
   tattttacca ctccctatca gtgatagaga aaagtgaaat gaatagttcg acaaaaatct     120
   agaaataatt ttgtttaact ttaagaagga gatatacaaa tgaagttcgg cctgttcttc     180
   ctgaacttca tgaacagcaa gaggtccagc gaccaggtga tcgaggaaat gctggacacc     240
   gcccactacg tggaccagct gaagttcgac accctggccg tgtacgagaa ccacttcagc     300
   aacaacggcg tggtgggagc ccctctgaca gtggccggct tcctgctggg catgaccaag     360
   aacgccaagg tggccagcct gaaccacgtg atcaccaccc accatcctgt gcgggtggcc     420
   gaggaagcct gcctgctgga ccagatgagc gagggcagat tcgccttcgg cttcagcgac     480
   tgcgagaagt ccgccgacat gcggttcttc aacaggccca ccgacagcca gttccagctg     540
   ttcagcgagt gccacaagat catcaacgac gccttcacca ccggctactg ccaccccaac     600
   aacgacttct acagcttccc caagatcagc gtgaaccccc acgccttcac agaaggcggc     660
   cctgcccagt tcgtgaacgc tacaagcaaa gaggtggtgg aatgggccgc taagctgggc     720
   ctgcccctgg tgttcagatg ggacgacagc aacgcccaga ggaaagagta cgccggcctg     780
   taccacgaag tggcccaggc tcatggcgtg gacgtgtccc aggtccggca caagctgacc     840
   ctgctggtga accagaacgt ggacggcgag gccgctagag ccgaggctag ggtgtacctg     900
   gaagagttcg tgcgggagag ctacagcaac accgacttcg agcagaagat gggcgagctg     960
   ctgagcgaga acgccatcgg cacctacgag gaaagcaccc aggccgccag agtggccatc    1020
   gagtgctgtg gagccgccga cctgctgatg agcttcgaga gcatggaaga taaggcccag    1080
   cagagggccg tgatcgacgt ggtgcggaga gtgaccgacg cccgggagaa cgagatggac    1140
   gagaacctgg aacaggtgtc cggcatcatc ggcaacctga ggcacatggc cctggacatg    1200
   ggcaacgaga tcgacaccca gaacaggcag atcgacagga tcatggaaaa ggccgacagc    1260
   aacaagacca ggatcgacga ggccaaccag agggccacca agatgctggg aagcggcaag    1320
   aagttcggca acttcctgct gacctaccag ccccctgagc tgagccagac cgaagtgatg 1380
   aagagactgg tgaacctggg caaggccagc gagggctgtg gcttcgacac cgtgtggctg 1440
   ctggaacacc acttcaccga gttcggactg ctgggcaacc cttacgtggc cgctgcccat 1500
   ctgctgggcg ccaccgagac actgaacgtg ggcaccgccg ccattgtgct gcctacagcc 1560
   caccctgtgc ggcaggctga ggacgtgaac ctgctggatc agatgtccaa gggcaggttc 1620
   agattcggca tctgcagggg cctgtacgac aaggacttca gggtgttcgg caccgacatg 1680
   gacaacagca gggccctgat ggactgttgg tacgacctga tgaaggaagg cttcaacgag 1740
   ggctacattg ccgccgacaa cgagcacatc aagttcccta agatccagct gaatcccagc 1800
   gcctacacac agggcggagc ccctgtgtac gtggtggccg agagcgcctc tacaaccgag 1860
   tgggctgccg agagggccct gcccatgatc ctgagctgga tcatcaacac ccacgagaag 1920
   aaggcccagc tggacctgta caatgaggtg gccaccgagc acggctacga cgtgaccaag 1980
   atcgaccact gcctgagcta catcaccagc gtggaccacg actccaacag ggccaaggac 2040
   atctgcagga actttctggg ccattggtac gacagctacg tgaacgctac caagatcttc 2100
   gacgacagcg accagaccaa gggctacgac ttcaacaagg gacagtggag ggacttcgtg 2160
   ctgaagggcc acaaggacac caacagacgg atcgactaca gctacgagat caaccccgtg 2220
   ggcacacctg aggaatgtat cgccatcatc cagcaggaca tcgacgccac cggcatcgac 2280
   aacatctgct gcggcttcga ggccaacggc agcgaggaag agatcattgc cagcatgaag 2340
   ctgttccaga gcgacgtgat gccctacctg aaagagaagc agggtctcag cgcttggagc 2400
   cacccgcagt tcgaaaaata ataagcttga cctgtgaagt gaaaaatggc gcacattgtg 2460
   cgacattttt tttgtctgcc gtttaccgct actgcgtcac ggatctccac gcgccctgta 2520
   gcggcgcatt aagcgcggcg ggtgtggtgg ttacgcgcag cgtgaccgct acacttgcca 2580
   gcgccctagc gcccgctcct ttcgctttct tcccttcctt tctcgccacg ttcgccggct 2640
   ttccccgtca agctctaaat cgggggctcc ctttagggtt ccgatttagt gctttacggc 2700
   acctcgaccc caaaaaactt gattagggtg atggttcacg tagtgggcca tcgccctgat 2760
   agacggtttt tcgccctttg acgttggagt ccacgttctt taatagtgga ctcttgttcc 2820
   aaactggaac aacactcaac cctatctcgg tctattcttt tgatttataa gggattttgc 2880
   cgatttcggc ctattggtta aaaaatgagc tgatttaaca aaaatttaac gcgaatttta 2940
   acaaaatatt aacgcttaca atttcaggtg gcacttttcg gggaaatgtg cgcggaaccc 3000
   ctatttgttt atttttctaa atacattcaa atatgtatcc gctcatgaga caataaccct 3060
   gataaatgct tcaataatat tgaaaaagga agagtatgag tattcaacat ttccgtgtcg 3120
   cccttattcc cttttttgcg gcattttgcc ttcctgtttt tgctcaccca gaaacgctgg 3180
   tgaaagtaaa agatgctgaa gatcagttgg gtgcacgagt gggttacatc gaactggatc 3240
   tcaacagcgg taagatcctt gagagttttc gccccgaaga acgttttcca atgatgagca 3300
   cttttaaagt tctgctatgt ggcgcggtat tatcccgtat tgacgccggg caagagcaac 3360
   tcggtcgccg catacactat tctcagaatg acttggttga gtactcacca gtcacagaaa 3420
   agcatcttac ggatggcatg acagtaagag aattatgcag tgctgccata accatgagtg 3480
   ataacactgc ggccaactta cttctgacaa cgatcggagg accgaaggag ctaaccgctt 3540
   ttttgcacaa catgggggat catgtaactc gccttgatcg ttgggaaccg gagctgaatg 3600
   aagccatacc aaacgacgag cgtgacacca cgatgcctgt agcaatggca acaacgttgc 3660
   gcaaactatt aactggcgaa ctacttactc tagcttcccg gcaacaattg atagactgga 3720
   tggaggcgga taaagttgca ggaccacttc tgcgctcggc ccttccggct ggctggttta 3780
   ttgctgataa atctggagcc ggtgagcgtg gctctcgcgg tatcattgca gcactggggc 3840
   cagatggtaa gccctcccgt atcgtagtta tctacacgac ggggagtcag gcaactatgg 3900
   atgaacgaaa tagacagatc gctgagatag gtgcctcact gattaagcat tggtaggaat 3960
   taatgatgtc tcgtttagat aaaagtaaag tgattaacag cgcattagag ctgcttaatg 4020
   aggtcggaat cgaaggttta acaacccgta aactcgccca gaagctaggt gtagagcagc 4080
   ctacattgta ttggcatgta aaaaataagc gggctttgct cgacgcctta gccattgaga 4140
   tgttagatag gcaccatact cacttttgcc ctttagaagg ggaaagctgg caagattttt 4200
   tacgtaataa cgctaaaagt tttagatgtg ctttactaag tcatcgcgat ggagcaaaag 4260
   tacatttagg tacacggcct acagaaaaac agtatgaaac tctcgaaaat caattagcct 4320
   ttttatgcca acaaggtttt tcactagaga atgcattata tgcactcagc gcagtggggc 4380
   attttacttt aggttgcgta ttggaagatc aagagcatca agtcgctaaa gaagaaaggg 4440
   aaacacctac tactgatagt atgccgccat tattacgaca agctatcgaa ttatttgatc 4500
   accaaggtgc agagccagcc ttcttattcg gccttgaatt gatcatatgc ggattagaaa 4560
   aacaacttaa atgtgaaagt gggtcttaaa agcagcataa cctttttccg tgatggtaac 4620
   ttcactagtt taaaaggatc taggtgaaga tcctttttga taatctcatg accaaaatcc 4680
   cttaacgtga gttttcgttc cactgagcgt cagaccccgt agaaaagatc aaaggatctt 4740
   cttgagatcc tttttttctg cgcgtaatct gctgcttgca aacaaaaaaa ccaccgctac 4800
   cagcggtggt ttgtttgccg gatcaagagc taccaactct ttttccgaag gtaactggct 4860
   tcagcagagc gcagatacca aatactgtcc ttctagtgta gccgtagtta ggccaccact 4920
   tcaagaactc tgtagcaccg cctacatacc tcgctctgct aatcctgtta ccagtggctg 4980
   ctgccagtgg cgataagtcg tgtcttaccg ggttggactc aagacgatag ttaccggata 5040
   aggcgcagcg gtcgggctga acggggggtt cgtgcacaca gcccagcttg gagcgaacga 5100
   cctacaccga actgagatac ctacagcgtg agctatgaga aagcgccacg cttcccgaag 5160
   ggagaaaggc ggacaggtat ccggtaagcg gcagggtcgg aacaggagag cgcacgaggg 5220
   agcttccagg gggaaacgcc tggtatcttt atagtcctgt cgggtttcgc cacctctgac 5280
   ttgagcgtcg atttttgtga tgctcgtcag gggggcggag cctatggaaa aacgccagca 5340
   acgcggcctt tttacggttc ctggcctttt gctggccttt tgctcacatg acccgaca 5398
<210> 11
   <211> 2262
   <212> DNA
   <213> Artificial Sequence
<220>
   <223> LuxBA3 with C-terminal Streptavidin-tag
<400> 11
   atgaagttcg gcctgttctt cctgaacttc atgaacagca agaggtccag cgaccaggtg 60
   atcgaggaaa tgctggacac cgcccactac gtggaccagc tgaagttcga caccctggcc 120
   gtgtacgaga accacttcag caacaacggc gtggtgggag cccctctgac agtggccggc 180
   ttcctgctgg gcatgaccaa gaacgccaag gtggccagcc tgaaccacgt gatcaccacc 240
   caccatcctg tgcgggtggc cgaggaagcc tgcctgctgg accagatgag cgagggcaga 300
   ttcgccttcg gcttcagcga ctgcgagaag tccgccgaca tgcggttctt caacaggccc 360
   accgacagcc agttccagct gttcagcgag tgccacaaga tcatcaacga cgccttcacc 420
   accggctact gccaccccaa caacgacttc tacagcttcc ccaagatcag cgtgaacccc 480
   cacgccttca cagaaggcgg ccctgcccag ttcgtgaacg ctacaagcaa agaggtggtg 540
   gaatgggccg ctaagctggg cctgcccctg gtgttcagat gggacgacag caacgcccag 600
   aggaaagagt acgccggcct gtaccacgaa gtggcccagg ctcatggcgt ggacgtgtcc 660
   caggtccggc acaagctgac cctgctggtg aaccagaacg tggacggcga ggccgctaga 720
   gccgaggcta gggtgtacct ggaagagttc gtgcgggaga gctacagcaa caccgacttc 780
   gagcagaaga tgggcgagct gctgagcgag aacgccatcg gcacctacga ggaaagcacc 840
   caggccgcca gagtggccat cgagtgctgt ggagccgccg acctgctgat gagcttcgag 900
   agcatggaag ataaggccca gcagagggcc gtgatcgacg tggtgcggag agtgaccgac 960
   gcccgggaga acgagatgga cgagaacctg gaacaggtgt ccggcatcat cggcaacctg 1020
   aggcacatgg ccctggacat gggcaacgag atcgacaccc agaacaggca gatcgacagg 1080
   atcatggaaa aggccgacag caacaagacc aggatcgacg aggccaacca gagggccacc 1140
   aagatgctgg gaagcggcaa gaagttcggc aacttcctgc tgacctacca gccccctgag 1200
   ctgagccaga ccgaagtgat gaagagactg gtgaacctgg gcaaggccag cgagggctgt 1260
   ggcttcgaca ccgtgtggct gctggaacac cacttcaccg agttcggact gctgggcaac 1320
   ccttacgtgg ccgctgccca tctgctgggc gccaccgaga cactgaacgt gggcaccgcc 1380
   gccattgtgc tgcctacagc ccaccctgtg cggcaggctg aggacgtgaa cctgctggat 1440
   cagatgtcca agggcaggtt cagattcggc atctgcaggg gcctgtacga caaggacttc 1500
   agggtgttcg gcaccgacat ggacaacagc agggccctga tggactgttg gtacgacctg 1560
   atgaaggaag gcttcaacga gggctacatt gccgccgaca acgagcacat caagttccct 1620
   aagatccagc tgaatcccag cgcctacaca cagggcggag cccctgtgta cgtggtggcc 1680
   gagagcgcct ctacaaccga gtgggctgcc gagaggggcc tgcccatgat cctgagctgg 1740
   atcatcaaca cccacgagaa gaaggcccag ctggacctgt acaatgaggt ggccaccgag 1800
   cacggctacg acgtgaccaa gatcgaccac tgcctgagct acatcaccag cgtggaccac 1860
   gactccaaca gggccaagga catctgcagg aactttctgg gccattggta cgacagctac 1920
   gtgaacgcta ccaagatctt cgacgacagc gaccagacca agggctacga cttcaacaag 1980
   ggacagtgga gggacttcgt gctgaagggc cacaaggaca ccaacagacg gatcgactac 2040
   agctacgaga tcaaccccgt gggcacacct gaggaatgta tcgccatcat ccagcaggac 2100
   atcgacgcca ccggcatcga caacatctgc tgcggcttcg aggccaacgg cagcgaggaa 2160
   gagatcattg ccagcatgaa gctgttccag agcgacgtga tgccctacct gaaagagaag 2220
   cagggtctca gcgcttggag ccacccgcag ttcgaaaaat aa 2262
<210> 12
   <211> 2937
   <212> DNA
   <213> Artificial Sequence
<220>
   <223> LuxBA3 with N-terminal glutathione-S-transferase and C-terminal Streptavidin-tag
<400> 12
   atgtccccta tactaggtta ttggaaaatt aagggccttg tgcaacccac tcgacttctt 60
   ttggaatatc ttgaagaaaa atatgaagag catttgtatg agcgcgatga aggtgataaa 120
   tggcgaaaca aaaagtttga attgggtttg gagtttccca atcttcctta ttatattgat 180
   ggtgatgtta aattaacaca gtctatggcc atcatacgtt atatagctga caagcacaac 240
   atgttgggtg gttgtccaaa agagcgtgca gagatttcaa tgcttgaagg agcggttttg 300
   gatattagat acggtgtttc gagaattgca tatagtaaag actttgaaac tctcaaagtt 360
   gattttctta gcaagctacc tgaaatgctg aaaatgttcg aagatcgttt atgtcataaa 420
   acatatttaa atggtgatca tgtaacccat cctgacttca tgttgtatga cgctcttgat 480
   gttgttttat acatggaccc aatgtgcctg gatgcgttcc caaaattagt ttgttttaaa 540
   aaacgtattg aagctatccc acaaattgat aagtacttga aatccagcaa gtatatagca 600
   tggcctttgc agggctggca agccacgttt ggtggtggcg accatcctcc aaaatcggat 660
   ctgatcgaag gtcggatgaa gttcggcctg ttcttcctga acttcatgaa cagcaagagg 720
   tccagcgacc aggtgatcga ggaaatgctg gacaccgccc actacgtgga ccagctgaag 780
   ttcgacaccc tggccgtgta cgagaaccac ttcagcaaca acggcgtggt gggagcccct 840
   ctgacagtgg ccggcttcct gctgggcatg accaagaacg ccaaggtggc cagcctgaac 900
   cacgtgatca ccacccacca tcctgtgcgg gtggccgagg aagcctgcct gctggaccag 960
   atgagcgagg gcagattcgc cttcggcttc agcgactgcg agaagtccgc cgacatgcgg 1020
   ttcttcaaca ggcccaccga cagccagttc cagctgttca gcgagtgcca caagatcatc 1080
   aacgacgcct tcaccaccgg ctactgccac cccaacaacg acttctacag cttccccaag 1140
   atcagcgtga acccccacgc cttcacagaa ggcggccctg cccagttcgt gaacgctaca 1200
   agcaaagagg tggtggaatg ggccgctaag ctgggcctgc ccctggtgtt cagatgggac 1260
   gacagcaacg cccagaggaa agagtacgcc ggcctgtacc acgaagtggc ccaggctcat 1320
   ggcgtggacg tgtcccaggt ccggcacaag ctgaccctgc tggtgaacca gaacgtggac 1380
   ggcgaggccg ctagagccga ggctagggtg tacctggaag agttcgtgcg ggagagctac 1440
   agcaacaccg acttcgagca gaagatgggc gagctgctga gcgagaacgc catcggcacc 1500
   tacgaggaaa gcacccaggc cgccagagtg gccatcgagt gctgtggagc cgccgacctg 1560
   ctgatgagct tcgagagcat ggaagataag gcccagcaga gggccgtgat cgacgtggtg 1620
   cggagagtga ccgacgcccg ggagaacgag atggacgaga acctggaaca ggtgtccggc 1680
   atcatcggca acctgaggca catggccctg gacatgggca acgagatcga cacccagaac 1740
   aggcagatcg acaggatcat ggaaaaggcc gacagcaaca agaccaggat cgacgaggcc 1800
   aaccagaggg ccaccaagat gctgggaagc ggcaagaagt tcggcaactt cctgctgacc 1860
   taccagcccc ctgagctgag ccagaccgaa gtgatgaaga gactggtgaa cctgggcaag 1920
   gccagcgagg gctgtggctt cgacaccgtg tggctgctgg aacaccactt caccgagttc 1980
   ggactgctgg gcaaccctta cgtggccgct gcccatctgc tgggcgccac cgagacactg 2040
   aacgtgggca ccgccgccat tgtgctgcct acagcccacc ctgtgcggca ggctgaggac 2100
   gtgaacctgc tggatcagat gtccaagggc aggttcagat tcggcatctg caggggcctg 2160
   tacgacaagg acttcagggt gttcggcacc gacatggaca acagcagggc cctgatggac 2220
   tgttggtacg acctgatgaa ggaaggcttc aacgagggct acattgccgc cgacaacgag 2280
   cacatcaagt tccctaagat ccagctgaat cccagcgcct acacacaggg cggagcccct 2340
   gtgtacgtgg tggccgagag cgcctctaca accgagtggg ctgccgagag gggcctgccc 2400
   atgatcctga gctggatcat caacacccac gagaagaagg cccagctgga cctgtacaat 2460
   gaggtggcca ccgagcacgg ctacgacgtg accaagatcg accactgcct gagctacatc     2520
   accagcgtgg accacgactc caacagggcc aaggacatct gcaggaactt tctgggccat     2580
   tggtacgaca gctacgtgaa cgctaccaag atcttcgacg acagcgacca gaccaagggc     2640
   tacgacttca acaagggaca gtggagggac ttcgtgctga agggccacaa ggacaccaac     2700
   agacggatcg actacagcta cgagatcaac cccgtgggca cacctgagga atgtatcgcc     2760
   atcatccagc aggacatcga cgccaccggc atcgacaaca tctgctgcgg cttcgaggcc     2820
   aacggcagcg aggaagagat cattgccagc atgaagctgt tccagagcga cgtgatgccc     2880
   tacctgaaag agaagcaggg tctcagcgct tggagccacc cgcagttcga aaaataa        2937
<210> 13
   <211> 2307
   <212> DNA
   <213> Artificial Sequence
<220>
   <223> LuxBA3 with N-terminal 9xHistidin and C-terminal Streptavidin-tag
<400> 13
   atgggccatc atcatcatca tcaccaccat cacatcgaag gtcggatgaa gttcggcctg      60
   ttcttcctga acttcatgaa cagcaagagg tccagcgacc aggtgatcga ggaaatgctg     120
   gacaccgccc actacgtgga ccagctgaag ttcgacaccc tggccgtgta cgagaaccac     180
   ttcagcaaca acggcgtggt gggagcccct ctgacagtgg ccggcttcct gctgggcatg     240
   accaagaacg ccaaggtggc cagcctgaac cacgtgatca ccacccacca tcctgtgcgg     300
   gtggccgagg aagcctgcct gctggaccag atgagcgagg gcagattcgc cttcggcttc     360
   agcgactgcg agaagtccgc cgacatgcgg ttcttcaaca ggcccaccga cagccagttc     420
   cagctgttca gcgagtgcca caagatcatc aacgacgcct tcaccaccgg ctactgccac     480
   cccaacaacg acttctacag cttccccaag atcagcgtga acccccacgc cttcacagaa     540
   ggcggccctg cccagttcgt gaacgctaca agcaaagagg tggtggaatg ggccgctaag     600
   ctgggcctgc ccctggtgtt cagatgggac gacagcaacg cccagaggaa agagtacgcc     660
   ggcctgtacc acgaagtggc ccaggctcat ggcgtggacg tgtcccaggt ccggcacaag     720
   ctgaccctgc tggtgaacca gaacgtggac ggcgaggccg ctagagccga ggctagggtg     780
   tacctggaag agttcgtgcg ggagagctac agcaacaccg acttcgagca gaagatgggc     840
   gagctgctga gcgagaacgc catcggcacc tacgaggaaa gcacccaggc cgccagagtg     900
   gccatcgagt gctgtggagc cgccgacctg ctgatgagct tcgagagcat ggaagataag     960
   gcccagcaga gggccgtgat cgacgtggtg cggagagtga ccgacgcccg ggagaacgag    1020
   atggacgaga acctggaaca ggtgtccggc atcatcggca acctgaggca catggccctg    1080
   gacatgggca acgagatcga cacccagaac aggcagatcg acaggatcat ggaaaaggcc 1140
   gacagcaaca agaccaggat cgacgaggcc aaccagaggg ccaccaagat gctgggaagc 1200
   ggcaagaagt tcggcaactt cctgctgacc taccagcccc ctgagctgag ccagaccgaa 1260
   gtgatgaaga gactggtgaa cctgggcaag gccagcgagg gctgtggctt cgacaccgtg 1320
   tggctgctgg aacaccactt caccgagttc ggactgctgg gcaaccctta cgtggccgct 1380
   gcccatctgc tgggcgccac cgagacactg aacgtgggca ccgccgccat tgtgctgcct 1440
   acagcccacc ctgtgcggca ggctgaggac gtgaacctgc tggatcagat gtccaagggc 1500
   aggttcagat tcggcatctg caggggcctg tacgacaagg acttcagggt gttcggcacc 1560
   gacatggaca acagcagggc cctgatggac tgttggtacg acctgatgaa ggaaggcttc 1620
   aacgagggct acattgccgc cgacaacgag cacatcaagt tccctaagat ccagctgaat 1680
   cccagcgcct acacacaggg cggagcccct gtgtacgtgg tggccgagag cgcctctaca 1740
   accgagtggg ctgccgagag gggcctgccc atgatcctga gctggatcat caacacccac 1800
   gagaagaagg cccagctgga cctgtacaat gaggtggcca ccgagcacgg ctacgacgtg 1860
   accaagatcg accactgcct gagctacatc accagcgtgg accacgactc caacagggcc 1920
   aaggacatct gcaggaactt tctgggccat tggtacgaca gctacgtgaa cgctaccaag 1980
   atcttcgacg acagcgacca gaccaagggc tacgacttca acaagggaca gtggagggac 2040
   ttcgtgctga agggccacaa ggacaccaac agacggatcg actacagcta cgagatcaac 2100
   cccgtgggca cacctgagga atgtatcgcc atcatccagc aggacatcga cgccaccggc 2160
   atcgacaaca tctgctgcgg cttcgaggcc aacggcagcg aggaagagat cattgccagc 2220
   atgaagctgt tccagagcga cgtgatgccc tacctgaaag agaagcaggg tctcagcgct 2280
   tggagccacc cgcagttcga aaaataa 2307
<210> 14
   <211> 7555
   <212> DNA
   <213> Artificial Sequence
<220>
   <223> ligation of LuxBA3 into pTZ_E47 vector
<400> 14
   tggcgaatgg gacgcgccct gtagcggcgc attaagcgcg gcgggtgtgg tggttacgcg      60
   cagcgtgacc gctacacttg ccagcgccct agcgcccgct cctttcgctt tcttcccttc     120
   ctttctcgcc acgttcgccg gctttccccg tcaagctcta aatcgggggc tccctttagg     180
   gttccgattt agtgctttac ggcacctcga ccccaaaaaa cttgattagg gtgatggttc     240
   acgtagtggg ccatcgccct gatagacggt ttttcgccct ttgacgttgg agtccacgtt     300
   ctttaatagt ggactcttgt tccaaactgg aacaacactc aaccctatct cggtctattc     360
   ttttgattta taagggattt tgccgatttc ggcctattgg ttaaaaaatg agctgattta     420
   acaaaaattt aacgcgaatt ttaacaaaat attaacgttt acaatttcag gtggcacttt     480
   tcggggaaat gtgcgcggaa cccctatttg tttatttttc taaatacatt caaatatgta     540
   tccgctcatg aattaattct tagaaaaact catcgagcat caaatgaaac tgcaatttat     600
   tcatatcagg attatcaata ccatattttt gaaaaagccg tttctgtaat gaaggagaaa     660
   actcaccgag gcagttccat aggatggcaa gatcctggta tcggtctgcg attccgactc     720
   gtccaacatc aatacaacct attaatttcc cctcgtcaaa aataaggtta tcaagtgaga     780
   aatcaccatg agtgacgact gaatccggtg agaatggcaa aagtttatgc atttctttcc     840
   agacttgttc aacaggccag ccattacgct cgtcatcaaa atcactcgca tcaaccaaac     900
   cgttattcat tcgtgattgc gcctgagcga gacgaaatac gcgatcgctg ttaaaaggac     960
   aattacaaac aggaatcgaa tgcaaccggc gcaggaacac tgccagcgca tcaacaatat    1020
   tttcacctga atcaggatat tcttctaata cctggaatgc tgttttcccg gggatcgcag    1080
   tggtgagtaa ccatgcatca tcaggagtac ggataaaatg cttgatggtc ggaagaggca    1140
   taaattccgt cagccagttt agtctgacca tctcatctgt aacatcattg gcaacgctac    1200
   ctttgccatg tttcagaaac aactctggcg catcgggctt cccatacaat cgatagattg    1260
   tcgcacctga ttgcccgaca ttatcgcgag cccatttata cccatataaa tcagcatcca    1320
   tgttggaatt taatcgcggc ctagagcaag acgtttcccg ttgaatatgg ctcataacac    1380
   cccttgtatt actgtttatg taagcagaca gttttattgt tcatgaccaa aatcccttaa    1440
   cgtgagtttt cgttccactg agcgtcagac cccgtagaaa agatcaaagg atcttcttga    1500
   gatccttttt ttctgcgcgt aatctgctgc ttgcaaacaa aaaaaccacc gctaccagcg    1560
   gtggtttgtt tgccggatca agagctacca actctttttc cgaaggtaac tggcttcagc    1620
   agagcgcaga taccaaatac tgtccttcta gtgtagccgt agttaggcca ccacttcaag    1680
   aactctgtag caccgcctac atacctcgct ctgctaatcc tgttaccagt ggctgctgcc    1740
   agtggcgata agtcgtgtct taccgggttg gactcaagac gatagttacc ggataaggcg    1800
   cagcggtcgg gctgaacggg gggttcgtgc acacagccca gcttggagcg aacgacctac    1860
   accgaactga gatacctaca gcgtgagcta tgagaaagcg ccacgcttcc cgaagggaga    1920
   aaggcggaca ggtatccggt aagcggcagg gtcggaacag gagagcgcac gagggagctt    1980
   ccagggggaa acgcctggta tctttatagt cctgtcgggt ttcgccacct ctgacttgag    2040
   cgtcgatttt tgtgatgctc gtcagggggg cggagcctat ggaaaaacgc cagcaacgcg    2100
   gcctttttac ggttcctggc cttttgctgg ccttttgctc acatgttctt tcctgcgtta    2160
   tcccctgatt ctgtggataa ccgtattacc gcctttgagt gagctgatac cgctcgccgc    2220
   agccgaacga ccgagcgcag cgagtcagtg agcgaggaag cggaagagcg cctgatgcgg 2280
   tattttctcc ttacgcatct gtgcggtatt tcacaccgca tatatggtgc actctcagta 2340
   caatctgctc tgatgccgca tagttaagcc agtatacact ccgctatcgc tacgtgactg 2400
   ggtcatggct gcgccccgac acccgccaac acccgctgac gcgccctgac gggcttgtct 2460
   gctcccggca tccgcttaca gacaagctgt gaccgtctcc gggagctgca tgtgtcagag 2520
   gttttcaccg tcatcaccga aacgcgcgag gcagctgcgg taaagctcat cagcgtggtc 2580
   gtgaagcgat tcacagatgt ctgcctgttc atccgcgtcc agctcgttga gtttctccag 2640
   aagcgttaat gtctggcttc tgataaagcg ggccatgtta agggcggttt tttcctgttt 2700
   ggtcactgat gcctccgtgt aagggggatt tctgttcatg ggggtaatga taccgatgaa 2760
   acgagagagg atgctcacga tacgggttac tgatgatgaa catgcccggt tactggaacg 2820
   ttgtgagggt aaacaactgg cggtatggat gcggcgggac cagagaaaaa tcactcaggg 2880
   tcaatgccag cgcttcgtta atacagatgt aggtgttcca cagggtagcc agcagcatcc 2940
   tgcgatgcag atccggaaca taatggtgca gggcgctgac ttccgcgttt ccagacttta 3000
   cgaaacacgg aaaccgaaga ccattcatgt tgttgctcag gtcgcagacg ttttgcagca 3060
   gcagtcgctt cacgttcgct cgcgtatcgg tgattcattc tgctaaccag taaggcaacc 3120
   ccgccagcct agccgggtcc tcaacgacag gagcacgatc atgcgcaccc gtggggccgc 3180
   catgccggcg ataatggcct gcttctcgcc gaaacgtttg gtggcgggac cagtgacgaa 3240
   ggcttgagcg agggcgtgca agattccgaa taccgcaagc gacaggccga tcatcgtcgc 3300
   gctccagcga aagcggtcct cgccgaaaat gacccagagc gctgccggca cctgtcctac 3360
   gagttgcatg ataaagaaga cagtcataag tgcggcgacg atagtcatgc cccgcgccca 3420
   ccggaaggag ctgactgggt tgaaggctct caagggcatc ggtcgagatc ccggtgccta 3480
   atgagtgagc taacttacat taattgcgtt gcgctcactg cccgctttcc agtcgggaaa 3540
   cctgtcgtgc cagctgcatt aatgaatcgg ccaacgcgcg gggagaggcg gtttgcgtat 3600
   tgggcgccag ggtggttttt cttttcacca gtgagacggg caacagctga ttgcccttca 3660
   ccgcctggcc ctgagagagt tgcagcaagc ggtccacgct ggtttgcccc agcaggcgaa 3720
   aatcctgttt gatggtggtt aacggcggga tataacatga gctgtcttcg gtatcgtcgt 3780
   atcccactac cgagatatcc gcaccaacgc gcagcccgga ctcggtaatg gcgcgcattg 3840
   cgcccagcgc catctgatcg ttggcaacca gcatcgcagt gggaacgatg ccctcattca 3900
   gcatttgcat ggtttgttga aaaccggaca tggcactcca gtcgccttcc cgttccgcta 3960
   tcggctgaat ttgattgcga gtgagatatt tatgccagcc agccagacgc agacgcgccg 4020
   agacagaact taatgggccc gctaacagcg cgatttgctg gtgacccaat gcgaccagat 4080
   gctccacgcc cagtcgcgta ccgtcttcat gggagaaaat aatactgttg atgggtgtct 4140
   ggtcagagac atcaagaaat aacgccggaa cattagtgca ggcagcttcc acagcaatgg 4200
   catcctggtc atccagcgga tagttaatga tcagcccact gacgcgttgc gcgagaagat 4260
   tgtgcaccgc cgctttacag gcttcgacgc cgcttcgttc taccatcgac accaccacgc 4320
   tggcacccag ttgatcggcg cgagatttaa tcgccgcgac aatttgcgac ggcgcgtgca 4380
   gggccagact ggaggtggca acgccaatca gcaacgactg tttgcccgcc agttgttgtg 4440
   ccacgcggtt gggaatgtaa ttcagctccg ccatcgccgc ttccactttt tcccgcgttt 4500
   tcgcagaaac gtggctggcc tggttcacca cgcgggaaac ggtctgataa gagacaccgg 4560
   catactctgc gacatcgtat aacgttactg gtttcacatt caccaccctg aattgactct 4620
   cttccgggcg ctatcatgcc ataccgcgaa aggttttgcg ccattcgatg gtgtccggga 4680
   tctcgacgct ctcccttatg cgactcctgc attaggaagc agcccagtag taggttgagg 4740
   ccgttgagca ccgccgccgc aaggaatggt gcatgcaagg agatggcgcc caacagtccc 4800
   ccggccacgg ggcctgccac catacccacg ccgaaacaag cgctcatgag cccgaagtgg 4860
   cgagcccgat cttccccatc ggtgatgtcg gcgatatagg cgccagcaac cgcacctgtg 4920
   gcgccggtga tgccggccac gatgcgtccg gcgtagagga tcgagatctc gatcccgcga 4980
   aattaatacg actcactata ggggaattgt gagcggataa caattcccct ctagaaataa 5040
   ttttgtttaa ctttaagaag gagatatacc atgggccatc atcatcatca tcaccaccat 5100
   cacatcgaag gtcggatgaa gttcggcctg ttcttcctga acttcatgaa cagcaagagg 5160
   tccagcgacc aggtgatcga ggaaatgctg gacaccgccc actacgtgga ccagctgaag 5220
   ttcgacaccc tggccgtgta cgagaaccac ttcagcaaca acggcgtggt gggagcccct 5280
   ctgacagtgg ccggcttcct gctgggcatg accaagaacg ccaaggtggc cagcctgaac 5340
   cacgtgatca ccacccacca tcctgtgcgg gtggccgagg aagcctgcct gctggaccag 5400
   atgagcgagg gcagattcgc cttcggcttc agcgactgcg agaagtccgc cgacatgcgg 5460
   ttcttcaaca ggcccaccga cagccagttc cagctgttca gcgagtgcca caagatcatc 5520
   aacgacgcct tcaccaccgg ctactgccac cccaacaacg acttctacag cttccccaag 5580
   atcagcgtga acccccacgc cttcacagaa ggcggccctg cccagttcgt gaacgctaca 5640
   agcaaagagg tggtggaatg ggccgctaag ctgggcctgc ccctggtgtt cagatgggac 5700
   gacagcaacg cccagaggaa agagtacgcc ggcctgtacc acgaagtggc ccaggctcat 5760
   ggcgtggacg tgtcccaggt ccggcacaag ctgaccctgc tggtgaacca gaacgtggac 5820
   ggcgaggccg ctagagccga ggctagggtg tacctggaag agttcgtgcg ggagagctac 5880
   agcaacaccg acttcgagca gaagatgggc gagctgctga gcgagaacgc catcggcacc 5940
   tacgaggaaa gcacccaggc cgccagagtg gccatcgagt gctgtggagc cgccgacctg 6000
   ctgatgagct tcgagagcat ggaagataag gcccagcaga gggccgtgat cgacgtggtg     6060
   cggagagtga ccgacgcccg ggagaacgag atggacgaga acctggaaca ggtgtccggc     6120
   atcatcggca acctgaggca catggccctg gacatgggca acgagatcga cacccagaac     6180
   aggcagatcg acaggatcat ggaaaaggcc gacagcaaca agaccaggat cgacgaggcc     6240
   aaccagaggg ccaccaagat gctgggaagc ggcaagaagt tcggcaactt cctgctgacc     6300
   taccagcccc ctgagctgag ccagaccgaa gtgatgaaga gactggtgaa cctgggcaag     6360
   gccagcgagg gctgtggctt cgacaccgtg tggctgctgg aacaccactt caccgagttc     6420
   ggactgctgg gcaaccctta cgtggccgct gcccatctgc tgggcgccac cgagacactg     6480
   aacgtgggca ccgccgccat tgtgctgcct acagcccacc ctgtgcggca ggctgaggac     6540
   gtgaacctgc tggatcagat gtccaagggc aggttcagat tcggcatctg caggggcctg     6600
   tacgacaagg acttcagggt gttcggcacc gacatggaca acagcagggc cctgatggac     6660
   tgttggtacg acctgatgaa ggaaggcttc aacgagggct acattgccgc cgacaacgag     6720
   cacatcaagt tccctaagat ccagctgaat cccagcgcct acacacaggg cggagcccct     6780
   gtgtacgtgg tggccgagag cgcctctaca accgagtggg ctgccgagag gggcctgccc     6840
   atgatcctga gctggatcat caacacccac gagaagaagg cccagctgga cctgtacaat     6900
   gaggtggcca ccgagcacgg ctacgacgtg accaagatcg accactgcct gagctacatc     6960
   accagcgtgg accacgactc caacagggcc aaggacatct gcaggaactt tctgggccat     7020
   tggtacgaca gctacgtgaa cgctaccaag atcttcgacg acagcgacca gaccaagggc     7080
   tacgacttca acaagggaca gtggagggac ttcgtgctga agggccacaa ggacaccaac     7140
   agacggatcg actacagcta cgagatcaac cccgtgggca cacctgagga atgtatcgcc     7200
   atcatccagc aggacatcga cgccaccggc atcgacaaca tctgctgcgg cttcgaggcc     7260
   aacggcagcg aggaagagat cattgccagc atgaagctgt tccagagcga cgtgatgccc     7320
   tacctgaaag agaagcaggg tctcagcgct tggagccacc cgcagttcga aaaataatag     7380
   gcgcgccgga tcctcgagca ccaccaccac caccactgag atccggctgc taacaaagcc     7440
   cgaaaggaag ctgagttggc tgctgccacc gctgagcaat aactagcata accccttggg     7500
   gcctctaaac gggtcttgag gggttttttg ctgaaaggag gaactatatc cggat          7555

## Description

The present invention is concerned with test systems for determining the activity of neurotoxin polypeptides. Specifically, it relates to a polynucleotide encoding a single chain luciferase fusion polypeptide comprising from the N-terminus to the C-terminus: (i) a LuxB subunit, (ii) a linker comprising a neurotoxin cleavage site, and (iii) a LuxA subunit, wherein said luciferase subunits LuxA and LuxB are from a bacterial luciferase, and a polypeptide encoded by said polynucleotide. Further provided in accordance with the invention are a vector and a host cell comprising said polynucleotide. Moreover, the present invention relates to a method for determining a proteolytically active neurotoxin polypeptide in a sample and a kit for carrying out said method.

Clostridium botulinum and Clostridium tetani produce highly potent neurotoxins, i.e. botulinum toxins (BoNTs) and tetanus toxin (TeNT), respectively. These Clostridial neurotoxins specifically bind to neuronal cells and disrupt neurotransmitter release. Each toxin is synthesized as an inactive unprocessed approximately 150 kDa single-chain protein. The posttranslational processing involves formation of disulfide bridges, and limited proteolysis (nicking) by bacterial protease(s). Active dichain neurotoxin consists of two chains, an N-terminal light chain of approx. 50 kDa and a heavy chain of approx. 100 kDa linked by a disulfide bond. Neurotoxins structurally consist of three domains, i.e. the catalytic light chain, the heavy chain encompassing the translocation domain (N-terminal half) and the receptor binding domain (C-terminal half), see Krieglstein 1990, Eur J Biochem 188, 39; Krieglstein 1991, Eur J Biochem 202, 41; Krieglstein 1994, J Protein Chem 13, 49.

Clostridium botulinum secretes seven antigenically distinct serotypes designated A to G of the BoNTs. All serotypes together with the related TeNT secreted by Clostridium tetani, are zinc (Zn2+)-dependent endoproteases that block synaptic exocytosis by cleaving SNARE proteins and, in particular in the case of BoNT/A, C or E, SNAP-25. BoNTs cause, inter alia, the flaccid muscular paralysis seen in botulism, see Fischer 2007, PNAS 104, 10447.

Despite its toxic effects, BoNTs have been used as a therapeutic agents in a large number of diseases. BoNT serotype A (BoNT/A) was approved for human use in the United States in 1989 for the treatment of strabism, blepharospasm, and other disorders. It is commercially available as a protein preparation, for example, under the tradename BOTOX (Allergan Inc) under the tradename DYSPORT (Ipsen Ltd). For therapeutic application the complex is injected directly into the muscle to be treated. At physiological pH, the toxin is released from the protein complex and the desired pharmacological effect takes place. An improved BoNT/A preparation being free of complexing proteins is available under the tradename XEOMIN (Merz Pharmaceuticals GmbH).

BoNTs, in principle, weaken voluntary muscle strength and are, therefore, effective therapeutic agents for the therapy of diseases such as strabism, focal dystonia, including cervical dystonia, and benign essential blepharospasm or spasticity. They have been further shown to relief hemifacial spasm, and focal spasticity, and moreover, to be effective in a wide range of other indications, such as gastrointestinal disorders, hyperhidrosis, and cosmetic wrinkle correction, see Jost 2007, Drugs 67, 669.

The determination of the biological activity is important as a safety measure, for quality control and for quantification purposes. The mouse LD50 assay is currently the only reliable assay for quantifying the biological activity of neurotoxins and for assessing their therapeutic potential and/or their toxicity. Said assay is also accepted for quality control purposes during manufacture of neurotoxin. In the mouse LD50 bioassay, lethal and sub-lethal concentrations of a sample containing the neurotoxin polypeptide have to be injected into at least 120 animals. The number of killed animals over an observation period of 72 hours allows determining the neurotoxin polypeptide concentration in the sample. Apparent drawbacks of this assay are the high number of animals which will be sacrificed and the high level of stress and pain for said animals during the test.

In vitro assays which have been proposed so far are based on determining SNAP-25 cleavage in a cell free system or on neurotoxin exposure to primary neurons. However, these assay are less reliable and/or do not take into account all of the desired neurotoxin functions. Thus, at present, the LD50 bioassay described above is the only reliable assay which is described in the monograph for BoNT/A in the European pharmacopeia. However, there is a need for a reliable assay for measuring neurotoxin activity which avoids the drawbacks of the LD50 bioassay. WO2005/038029 discloses a method for detecting botulinum neurotoxin, comprising contacting a sample with a beetle luciferase enzyme having a botulinum neurotoxin cleavage site inserted in its amino acid sequence, and detecting luciferase activity.

Therefore, the technical problem underlying the present invention could be seen in the provision of means and methods for complying with the aforementioned needs. The technical problem is solved by the embodiments characterized in the claims and herein described below.

The present invention relates to a polynucleotide encoding a single chain luciferase fusion polypeptide comprising from the N-terminus to the C-terminus: (i) a LuxB subunit, (ii) a linker comprising a neurotoxin cleavage site, and (iii) a LuxA subunit, wherein said luciferase LuxA and LuxB are from a bacterial luciferase.

The term "polynucleotide" as used herein refers to single- or double-stranded DNA molecules as well as to RNA molecules. Encompassed by the said term is genomic DNA, cDNA, hnRNA, mRNA as well as all naturally occurring or artificially modified derivatives of such molecular species. The polynucleotide may be in an aspect a linear or circular molecule. Moreover, in addition to the nucleic acid sequences encoding the polypeptide of the present invention, a polynucleotide of the present invention may comprise additional sequences required for proper transcription and/or translation such as 5'- or 3'-UTR sequences. The nucleic acid sequences encoding the polypeptide of the present invention can be derived from the amino acid sequence envisaged for the single chain luciferase fusion polypeptide of the invention by a skilled artisan without further ado. In light of the degeneracy of the genetic code, optimized codons may be used in the nucleic acid sequences encoding the single chain luciferase fusion polypeptide in the polynucleotide of the present invention. Thereby, optimal expression in, e.g., a host cell of the present invention can be achieved.

The term "single chain luciferase fusion polypeptide" as used herein refers to a polypeptide comprising within a single polypeptide chain a LuxB subunit of a luciferase as well as a LuxA subunit. The said subunits are separated by a linker that comprises a neurotoxin cleavage site which is specifically recognized and cleaved by a proteolytically active neurotoxin polypeptide as referred to elsewhere herein. The single chain luciferase fusion polypeptide, in an aspect, has the LuxB and LuxA subunits arranged in a manner which prevents or reduces the formation of a biologically active luciferase holoenzyme when the subunits are present in the single chain luciferase fusion polypeptide. In an aspect, a biologically active luciferase holoenzyme is, thus, formed only or formed more efficiently after cleavage of the single chain luciferase fusion polypeptide by the neurotoxin protease. If the enzymatic activity, in an aspect, is prevented, essentially no activity shall occur when the single chain luciferase fusion polypeptide is in the single chain state. A reduced enzymatic activity as referred to herein, in an aspect, means a statistically significant reduced activity. In an aspect, the reduction of the activity in the single chain state versus the holoenzyme formed after cleavage is at least 10%, at least 20%, at least 30%, at least 40%, at least 50%, at least 60%, at least 70% or at least 80%.

In an aspect, the LuxB and LuxA subunits in the single chain luciferase fusion polypeptide are arranged in a manner which prevents or reduces the formation of a biologically active luciferase holoenzyme in that the order of the subunits in the single chain luciferase fusion polypeptide is from N to C-terminus: (i) a LuxB subunit, (ii) a linker comprising a neurotoxin cleavage site, and (iii) a LuxA subunit. Such an order of the subunits has been described to efficiently prevent luciferase activity in a single chain fusion protein (see Olsson 1989, Gene 81: 335-347).

In another aspect, the LuxB and LuxA subunits in the single chain luciferase fusion polypeptide are separated in addition to the linker by further polypeptide elements which prevent or reduce the capability of the subunits to interact with each other. This can be achieved, in an aspect, by inserting ankyrin repeats into the single chain luciferase fusion polypeptide either between the LuxB subunit and the linker or between the LuxA subunit and the linker or both. Ankyrin repeats are known to reduce the flexibility of a polypeptide chain and, thus, will reduce the capability of the separated luciferase subunits in the single chain luciferase fusion polypeptide of the invention to interact with each other. Ankyrins are well known in the art and belong into the family of adaptor proteins mediating the attachment of transmembrane proteins and cytoskeletal proteins. In mammals, three different ankyrins are known, ANK1, ANK2 and ANK3, occurring in various splice forms. The ankyrins have an N-terminal domain comprising the so-called ankyrin repeats which are to be applied in accordance with the present invention (see Wetzel 2008, J Mol Biol. 376(1): 241-57; Michaely 1995, J Biol Chem. 270(52):31298-302; Batrukova 2000, Biochemistry (Mosc). 65(4):395-408.).

In another aspect, a prevention or reduction of the capability of the subunits to interact with each other can be achieved by inserting protein domains or proteins either between the LuxB subunit and the linker, between the LuxA subunit and the linker or both which due to their molecular size sterically interfere with the interaction of the subunits with each other. In an aspect, globular proteins or domains thereof can be applied for this purpose. In an aspect, the said globular protein or domain thereof is selected from the group consisting: glutathione S-transferase (GST), maltose binding protein, small ubiquitin-like modifier (SUMO) protein, green fluorescent protein (GFP), red fluorescent protein (RFP), yellow fluorescent protein (YFP), blue fluorescent protein (BFP), and the like. In another aspect, a fluorescent protein may be bound to the neurotoxin and/or luciferase polypeptides specified elsewhere herein, whereby said fluorescent protein e.g. alters light emission of said polypeptide and/or is be used as internal control to monitor expression, number of cells, loading control, and the like. In yet an aspect, the linker can be comprised in a globular protein or domain thereof as described before.

It will be understood that the neurotoxin cleavage site comprised in the linker shall be made available by the single chain luciferase fusion protein to a neurotoxin polypeptide such that the neurotoxin protease can recognize, bind to and cleave the single chain luciferase fusion polypeptide under suitable conditions. The skilled artisan is well aware of how a suitable arrangement within the single chain luciferase fusion polypeptide can be designed. Moreover, the single chain luciferase fusion protein can be tested for cleavage by proteolytically active neurotoxin polypeptide as described in the accompanying Examples. In an aspect, the single chain luciferase fusion polypeptide of the invention has an amino acid sequence as shown in any one of SEQ ID NOs: 1 to 3.

The term "luciferase" as used herein refers to enzymes belonging into a class of enzymes capable of catalyzing a light-emitting reaction. Luciferases occur naturally as firely or bacterial luciferases. Such luciferases as enzymatically (biologically) active holoenzymes are composed of different subunits. In an aspect, the luciferase referred to herein is a bacterial luciferase and the subunits to be inserted into the single chain luciferase fusion polypeptide are LuxA and LuxB. In yet another aspect, the said LuxA and/or LuxB subunits are from Vibrio fischeri or Vibrio harveyi. How to arrange such subunits in a singly chain luciferase fusion protein without additional linker according to the present invention is known in the art and described in Olsson 1989, loc cit.. The structure of the aforementioned luciferases and their subunits as well as nucleic acid sequences encoding them are well known in the art and describe, e.g., in Cohn 1985, J Biol Chem. 260(10):6139-46 and Johnston 1986, J Biol Chem. 261(11):4805-11. In an aspect, the LuxA subunit of Vibrio fischeri as referred to herein has an amino acid sequence as shown in SEQ ID NO: 4 or a variant thereof. In another aspect, the LuxB subunit of Vibrio fischeri has an amino acid sequence as shown in SEQ ID NO: 5 or a variant thereof. It will be understood that the present invention also encompasses variants of such specific amino acid or nucleic acid sequences encoding them as long as these variant sequences also allow for the formation of an enzymatically active luciferase holoenzyme. In an aspect, a sequence variant as used herein differs from the specific amino acid sequence or a specific nucleic acid sequence as specified before by one or more amino acid or nucleotide substitutions, additions and/or deletions. In another aspect, the said variant sequence is at least 50%, at least 60%, at least 70%, at least 80%, at least 90%, at least 95%, at least 98% or at least 99% identical to the specific sequence over the entire length or over at least a stretch of half of the length of the specific sequence. The term "identical" as used herein refers to sequence identity characterized by determining the number of identical amino acids between sequences wherein the sequences are aligned so that the highest order match is obtained. It can be calculated using published techniques or methods codified in computer programs such as, for example, BLASTP or FASTA (Altschul 1990, J Mol Biol 215, 403). The percent identity values are, in one aspect, calculated over the entire amino acid sequence or over a sequence stretch of at least 50% of the query sequence. A series of programs based on a variety of algorithms is available to the skilled worker for comparing different sequences. In this context, the algorithms of Needleman and Wunsch or Smith and Waterman give particularly reliable results. To carry out the sequence alignments, the program PileUp (Higgins 1989, CABIOS 5, 151) or the programs Gap and BestFit (Needleman 1970, J Mol Biol 48; 443; Smith 1981, Adv Appl Math 2, 482), which are part of the GCG software packet (Genetics Computer Group 1991, 575 Science Drive, Madison, Wisconsin, USA 53711), may be used. The sequence identity values recited above in percent (%) are to be determined, using the program GAP over the entire sequence region with the following settings: Gap Weight: 50, Length Weight: 3, Average Match: 10.000 and Average Mismatch: 0.000, which, unless otherwise specified, shall always be used as standard settings for sequence alignments.

The term "neurotoxin cleavage site" as used herein refers to a cleavage site which is recognized and cleaved by the endogenous protease of a neurotoxin polypeptide. Cleavage sites which are recognized by the neurotoxin proteases are well known in the art (see, e.g., EP 1 926 744 B1). In an aspect, the said neurotoxin cleavage site is selected from the group consisting of: a neurotoxin cleavage site from SNAP-25, a neurotoxin cleavage site from VAMP, a neurotoxin cleavage site from syntaxin, and the autocatalytic cleavage sites from neurotoxin polypeptides.

In principle, a neurotoxin cleavage site can be a cleavage site which naturally occurs in a substrate or which is an artificially designed cleavage site recognized and cleaved by the neurotoxin polypeptides protease. It will be understood that the properties of the neurotoxin cleavage site govern the kind of neurotoxin which can activate the polypeptide of the present invention. Neurotoxin polypeptides referred to herein, in an aspect, encompass BoNT/A, BoNT/B, BoNT/C1, BoNT/D, BoNT/E, BoNT/G, BoNT/F or TeNT all of which are well known in the art. For example, if a neurotoxin cleavage site is used which is specifically recognized and cleaved by BoNT/A, only the BoNT/A protease will be capable of activating the polypeptide of the present invention and, in particular, its luciferase activity, whereas if a neurotoxin cleavage site is used which is specifically recognized and cleaved by BoNT/E, only the BoNT/E protease will be capable of activating the polypeptide of the present invention and, in particular, its caspase activity. In an aspect of the invention, the neurotoxin cleavage site is cleaved by mature BoNTs. In yet another aspect, it is cleaved by muteins of BoNTs, in an aspect, by muteins comprising or consisting of the BoNT light chain exhibiting the BoNT protease activity.

A neurotoxin cleavage site recognized and cleaved by the BoNT/A protease, in an aspect of the invention, is derived from a protein that is sensitive to cleavage by BoNT/A. In an aspect, such a protein is human SNAP-25A or -25B or a homolog, paralog or ortholog thereof from rat, mouse, bovine, Danio, Carassius, Xenopus, Torpedo, Strongylocentrotus, Loligo, Lymnaea or Aplysia. Suitable cleavage sites derived from said proteins are disclosed in EP 1 926 744 B1.

A neurotoxin cleavage site recognized and cleaved by the BoNT/B protease, in an aspect of the invention, is derived from a protein that is sensitive to cleavage by BoNT/B. In an aspect, such a protein is human or mouse VAMP-1, VAMP-2 and VAMP-3/cellubrevin, bovine VAMP-2, rat VAMP-2 or VAMP-3, chicken VAMP-1, VAMP-2 or VAMP-3, Torpedo VAMP-1, Strongylocentrotus VAMP, Drosophila sybA, synB, synC, synD, or syn, Hirudo VAMP, Xenopus VAMP-2 or VAMP-3, Danio VAMP-1 or VAMP-2, Loligo VAMP, Lymnaea VAMP, Aplysia VAMP or Caenorhabditis SNB1-like or any ortholog, paralog or homolog thereof. Suitable cleavage sites derived from said proteins are disclosed in EP 1 926 744 B1 .

A neurotoxin cleavage site recognized and cleaved by the BoNT/C1 protease, in an aspect of the invention, is derived from a protein that is sensitive to cleavage by BoNT/C1. In an aspect, such a protein is human and mouse Syntaxin 1A, Syntaxin 1B1, Syntaxin 2-1, Syntaxin 2-2, Syntaxin 2-3, Syntaxin 3A or Syntaxin 1B2, bovine or rat Syntaxin 1A, Syntaxin 1B1 or Syntaxin 1B2, rat Syntaxin 2 or Rat syntaxin 3, mouse Syntaxin 1A, Syntaxin 1B1, Syntaxin 1B2, Syntaxin 2, Syntaxin 3A, Syntaxin 3B or Syntaxin 3C, chicken Syntaxin 1A or Syntaxin 2; Xenopus Syntaxin 1A or Syntaxin 1B, Danio Syntaxin 1A, Syntaxin 1B or Syntaxin 3, Torpedo Syntaxin 1A or Syntaxin 1B, Strongylocentrotus Syntaxin 1A or Syntaxin 1B, Drosophila Syntaxin 1A or Syntaxin 1B, Hirudo Syntaxin 1A or Syntaxin 1B, Loligo Syntaxin 1A or Syntaxin 1B, Lymnaea Syntaxin 1A or Syntaxin 1B or any ortholog, paralog or homolog thereof. Suitable cleavage sites derived from said proteins are disclosed in EP 1 926 744 B1.

A neurotoxin cleavage site recognized and cleaved by the BoNT/D protease, in an aspect of the invention, is derived from a protein that is sensitive to cleavage by BoNT/D. In an aspect, such a protein is human or mouse VAMP-1, VAMP-2 and VAMP-3/cellubrevin, bovine VAMP-2, rat VAMP-2 or VAMP-3, chicken VAMP-1, VAMP-2 or VAMP-3, Torpedo VAMP-1, Strongylocentrotus VAMP, Drosophila sybA, synB, synC, synD, or syn, Hirudo VAMP, Xenopus VAMP-2 or VAMP-3, Danio VAMP-1 or VAMP-2, Loligo VAMP, Lymnaea VAMP, Aplysia VAMP or Caenorhabditis SNB1-like or any ortholog, paralog or homolog thereof. Suitable cleavage sites derived from said proteins are disclosed in EP 1 926 744 B1.

A neurotoxin cleavage site recognized and cleaved by the BoNT/E protease, in an aspect of the invention, is derived from a protein that is sensitive to cleavage by BoNT/E. In an aspect, such a protein is, such a protein is human SNAP-25A or B or a homolog, paralog or ortholog thereof from rat, mouse, bovine, Danio, Carassius, Xenopus, Torpedo, Strongylocentrotus, Loligo, Lymnaea or Aplysia. Suitable cleavage sites derived from said proteins are disclosed in EP 1 926 744 B1.

A neurotoxin cleavage site recognized and cleaved by the BoNT/F protease, in an aspect of the invention, is derived from a protein that is sensitive to cleavage by BoNT/F. In an aspect, such a protein is, such a protein is human or mouse VAMP-1, VAMP-2 and VAMP-3/cellubrevin, bovine VAMP-2, rat VAMP-2 or VAMP-3, chicken VAMP-1, VAMP-2 or VAMP-3, Torpedo VAMP-1, Strongylocentrotus VAMP, Drosophila sybA, synB, synC, synD, or syn, Hirudo VAMP, Xenopus VAMP-2 or VAMP-3, Danio VAMP-1 or VAMP-2, Loligo VAMP, Lymnaea VAMP, Aplysia VAMP or Caenorhabditis SNB1-like or any ortholog, paralog or homolog thereof. Suitable cleavage sites derived from said proteins are disclosed in EP 1 926 744 B1.

A neurotoxin cleavage site recognized and cleaved by the BoNT/G protease, in an aspect of the invention, is derived from a protein that is sensitive to cleavage by BoNT/G. In an aspect, such a protein is, such a protein is human or mouse VAMP-1, VAMP-2 and VAMP-3/cellubrevin, bovine VAMP-2, rat VAMP-2 or VAMP-3, chicken VAMP-1, VAMP-2 or VAMP-3, Torpedo VAMP-1, Strongylocentrotus VAMP, Drosophila sybA, synB, synC, synD, or syn, Hirudo VAMP, Xenopus VAMP-2 or VAMP-3, Danio VAMP-1 or VAMP-2, Loligo VAMP, Lymnaea VAMP, Aplysia VAMP or Caenorhabditis SNB1-like or any ortholog, paralog or homolog thereof. Suitable cleavage sites derived from said proteins are disclosed in EP 1 926 744 B1.

A neurotoxin cleavage site recognized and cleaved by the TeNT protease, in an aspect of the invention, is derived from a protein that is sensitive to cleavage by TeNT. In an aspect, such a protein is human or mouse VAMP-1, VAMP-2 and VAMP-3/cellubrevin, bovine VAMP-2, rat VAMP-2 or VAMP-3, chicken VAMP-1, VAMP-2 or VAMP-3, Torpedo VAMP-1, Strongylocentrotus VAMP, Drosophila sybA, synB, synC, synD, or syn, Hirudo VAMP, Xenopus VAMP-2 or VAMP-3, Danio VAMP-1 or VAMP-2, Loligo VAMP, Lymnaea VAMP, Aplysia VAMP or Caenorhabditis SNB1-like or any ortholog, paralog or homolog thereof. Suitable cleavage sites derived from said proteins are disclosed in EP 1 926 744 B1.

A neurotoxin cleavage site recognized and cleaved by the BoNT proteases, in another aspect of the invention, is derived from the autocatalytic cleavage sites found in the BoNT proteins. In aspects, a neurotoxin cleavage site to be used in accordance with the present invention and which is derived from the autocatalytic cleavage site of a given BoNT or TeNT comprises at least 6, at least 8, at least 10 or at least 15 consecutive residues of including the BoNT/A residues 250Tyr-251Tyr, the BoNT/B residues 256Phe-257Phe, the BoNT/C1 residues 257Phe-258Tyr, the BoNT/D residues 257Phe-258Phe, the BoNT/E residues 239Pro-240Leu, the BoNT/F residues 254Pro-255Leu, the BoNT/G residues 256Phe-257Phe, the TeNT residues 259Ile-260Tyr, the BoNT/A residues Phe266-Gly267, the BoNT/B residues Phe272-Gly273, the BoNT/C1 residues Phe273-Gly274, the BoNT/D residues Phe273-Gly274, the BoNT/E residues Phe255-Gly256, the BoNT/F residues Phe270-Gly271, the BoNT/G residues Phe272-Gly273 or the TeNT residues Phe275-Gly276. Suitable cleavage sites derived from said BoNTs and TeNT are disclosed in EP 1 926 744 B1.

In yet an aspect of the invention, said neurotoxin cleavage site is a SNAP-25 derived cleavage site as shown in any one of SEQ ID NOs: 6 to 8.

Advantageously, the single chain luciferase fusion polypeptide of the present invention allows for the determination of the qualitative or quantitative protease activity of a given neurotoxin polypeptide in a cell culture system or in a cell free assay. Thus, expensive and unnecessary animal testing can be avoided or reduced thanks to the present invention. Moreover, the single chain luciferase fusion polypeptide can be applied in automated high throughput screening assays. The single chain luciferase fusion polypeptide also allows for establishing an assay in neuroblastoma cell lines rather than primary cells. Accordingly, the use of primary neurons as required in other cell based neurotoxin assays can be avoided. This is another advantage since the preparation of primary neurons is cumbersome and inefficient.

It is to be understood that the definitions and explanations of the terms made above apply mutatis mutandis for all aspects described in this specification in the following except as otherwise indicated.

The present invention also relates to a vector comprising the polynucleotide of the invention.

The term "vector", preferably, encompasses phage, plasmid, viral or retroviral vectors as well as artificial chromosomes, such as bacterial or yeast artificial chromosomes. Moreover, the term also relates to targeting constructs which allow for random or site-directed integration of the targeting construct into genomic DNA. Such target constructs, in an aspect, comprise DNA of sufficient length for either homologous or heterologous recombination as described in detail below. The vector encompassing the polynucleotides of the present invention, in an aspect, further comprises selectable markers for propagation and/or selection in a host cell. The vector may be incorporated into a host cell by various techniques well known in the art. For example, a plasmid vector can be introduced in a precipitate such as a calcium phosphate precipitate or rubidium chloride precipitate, or in a complex with a charged lipid or in carbon-based clusters, such as fullerens. Alternatively, a plasmid vector may be introduced by heat shock or electroporation techniques. Should the vector be a virus, it may be packaged in vitro using an appropriate packaging cell line prior to application to host cells. Retroviral vectors may be replication competent or replication defective. In the latter case, viral propagation generally will occur only in complementing host/cells.

Moreover, in an aspect of the invention, the polynucleotide is operatively linked to expression control sequences allowing expression in prokaryotic or eukaryotic host cells or isolated fractions thereof in the said vector. Thus, in an aspect, the vector is an expression vector. Expression of the polynucleotide comprises transcription of the polynucleotide into a translatable mRNA. Regulatory elements ensuring expression in host cells are well known in the art. In an aspect, they comprise regulatory sequences ensuring initiation of transcription and/or poly-A signals ensuring termination of transcription and stabilization of the transcript. Additional regulatory elements may include transcriptional as well as translational enhancers. Possible regulatory elements permitting expression in prokaryotic host cells comprise, e.g., the lac-, trp- or tac- promoter in E. coli, and examples for regulatory elements permitting expression in eukaryotic host cells are the AOX1- or the GAL1- promoter in yeast or the CMV-, SV40-, RSV-promoter (Rous sarcoma virus), CMV-enhancer, SV40-enhancer or a globin intron in mammalian and other animal cells. Moreover, inducible expression control sequences may be used in an expression vector encompassed by the present invention. Such inducible vectors may comprise tet or lac operator sequences or sequences inducible by heat shock or other environmental factors. Suitable expression control sequences are well known in the art. Beside elements which are responsible for the initiation of transcription such regulatory elements may also comprise transcription termination signals, such as the SV40-poly-A site or the tk-poly-A site, downstream of the polynucleotide. In this context, suitable expression vectors are known in the art such as Okayama-Berg cDNA expression vector pcDV1 (Pharmacia), pBluescript (Stratagene), pCDM8, pRc/CMV, pcDNA1, pcDNA3 (Invitrogen) or pSPORT1 (Invitrogen). Preferably, said vector is an expression vector and a gene transfer or targeting vector. Expression vectors derived from viruses such as retroviruses, vaccinia virus, adeno-associated virus, herpes viruses, or bovine papilloma virus, may be used for delivery of the polynucleotide or vector of the invention into a targeted cell population. Methods which are well known to those skilled in the art can be used to construct recombinant viral vectors; see, for example, the techniques described in Sambrook, Molecular Cloning A Laboratory Manual, Cold Spring Harbor Laboratory (1989) N.Y. and Ausubel, Current Protocols in Molecular Biology, Green Publishing Associates and Wiley Interscience, N.Y. (1994).

Moreover, the present invention relates to a host cell comprising the polynucleotide or the vector of the present invention.

The term "host cell" as used herein encompasses prokaryotic and eukaryotic host cells. In an aspect the host cell is a bacterial cell. In one aspect, the said bacterial host cell is an E.coli host cell. Such a bacterial host cell may be used, e.g., for reproduction of the polynucleotide or the vector of the present invention.

A eukaryotic host cell, in an aspect, is a cell which comprises the polypeptide and either the polynucleotide or the vector of the present invention wherein said polynucleotide or vector are expressed in the host cell in order to generate the polypeptide. The polynucleotide may be introduced into a host cell either transiently or stably. In an aspect, the eukaryotic host cell may be a cell of a eukaryotic host cell line which stably expresses the polynucleotide of the invention. In another aspect, the host cell is a eukaryotic host cell which has been transiently transfected with the polynucleotide or vector of the invention and which expresses the polynucleotide of the invention. In an aspect the host cell is a eukaryotic host cell which is capable of translocating a neurotoxin polypeptide into its cytoplasm. In an aspect, a cell capable of uptaking neurotoxin polypeptides can be a cell produces endogenously all necessary components for the neurotoxin polypeptide uptake. In an aspect, the said cell is a neuronal cell. In an aspect, the said cell is selected from the group consisting of: neuroblastoma cell lines, embryonic stem cells, and in an aspect non-human embryonic stem cells, induced pluripotent stem cells, and primary neurons, e.g. SH-SY5Y, SiMa, PC12, CHP134, LA-N-5, SK-N-BE(2), and the like. In another aspect, the said cell is a cell which has been genetically engineered to produce the components necessary for the neurotoxin polypeptide uptake. How such cells can be genetically engineered by molecular biology techniques is well known to the skilled person.

The present invention relates to a polypeptide encoded by the polynucleotide of the present invention.

The term "polypeptide" as used herein encompasses isolated or essentially purified polypeptides being essentially free of other host cell polypeptides. The term, in another aspect, includes polypeptide preparations comprising the polypeptide of the present invention and other proteins in addition. Moreover, the term includes, in an aspect, chemically modified polypeptides. Such modifications may be artificial modifications or naturally occurring modifications. The polypeptide of the present invention shall have the biological properties referred to above. The polypeptide of the invention, in an aspect, can be manufactured by chemical synthesis or recombinant molecular biology techniques well known for the skilled artisan. In an aspect, such a method of manufacturing the polypeptide of the invention comprises (a) culturing the host cell of the present invention described elsewhere herein in more detail and (b) obtaining from the said host cell the polypeptide of the present invention. In an aspect of this method, the polypeptide can be obtained by conventional purification techniques from a host cell lysate including affinity chromatography, ion exchange chromatography, size exclusion chromatography and/or preparative gel electrophoresis.

The present invention relates to a method for determining a proteolytically active neurotoxin polypeptide in a sample comprising:
a) contacting the host cell of or the polypeptide of the invention with a sample suspected to comprise said proteolytically active neurotoxin polypeptide under conditions which allow for proteolytic cleavage of the single chain luciferase fusion protein into separate LuxB and LuxA subunits;
b) allowing the said LuxB and LuxA subunit to form a biologically active luciferase; and
c) determining the said luciferase.

The method of the present invention can be assisted by automation. Specifically, in an aspect, step a) and /or b) may be assisted by robotic devices and automated reader systems for mixing compounds and measuring the luciferase activity. Suitable systems are known in the art and depend on the type of response to be determined. Moreover, the method may comprise additional steps pertaining to the sample preparation or generation of the polypeptide of the present invention.

The term "contacting" as used herein refers to bringing at least two different compounds in physical proximity as to allow physical and/or chemical interaction of said compounds. In the aforementioned method, the polypeptide according to the present invention is contacted with a sample suspected to comprise a biologically active neurotoxin polypeptide. The polypeptide shall be contacted for a time and under conditions sufficient to allow cleavage of the neurotoxin cleavage site in the polypeptide of the present invention by the neurotoxin polypeptide comprised by the sample. Contacting as used herein, in an aspect, occurs in a host cell of the present invention containing the polypeptide of the present invention. Thus, in an aspect, said polypeptide is comprised by a host cell and, in an aspect, the host cell of the present invention. The said time and conditions will dependent on the amount of neurotoxin polypeptide comprised by the sample as well as on the uptake of the neurotoxin polypeptide by the host cell. The person skilled in the art is well aware of which conditions need to be applied dependent on the host cell, kind of sample, and kind of neurotoxin which shall be determined. In another aspect, contacting occurs in a cell free system comprising the polypeptide of the invention as well as a substrate of the polypeptide of the present invention. The cell free system shall allow for measuring the activity of the polypeptide of the present invention, i.e. luciferase activity, upon contacting the system with a sample and, thus, allows for determining the neurotoxin protease activity in said sample.

In an aspect, said luciferase is determined by measuring the enzymatic conversion of a luciferase substrate. The latter one can be measured in an aspect by detecting the intensity of the light emitted during the conversion reaction. Suitable systems for measuring the light emission that occurs during the conversion reaction catalyzed by luciferases are well known in the art and commercially available. Moreover, suitable substrates which can be used for the luciferases are also well known and commercially available. In another aspect, however, the luciferase can be measured either by determining the amount of the formed LuxA ad/or B subunits or the formed holoenzyme. In an aspect, this determination is carried out by an antibody-based immunoassay using antibodies which specifically recognize a subunit or the holoenzyme, e.g., immunoblots or ELISA, or by SDS PAGE.

The term "sample" refers to a sample suspected to comprise neurotoxin polypeptide. The sample, in an aspect, is an aqueous solution. Such a sample may be a biological sample or may be a sample of an artificially generated aqueous solution. Such solutions, in an aspect, are obtained at different stages during neurotoxin manufacture, either for quality control and/or activity determination/specification purposes or for safety control. It is envisaged that the neurotoxin present in the said sample shall exhibit at least the neurotoxin protease activity. In another aspect, the neurotoxin is fully biologically active. In an aspect the said fully biologically active neurotoxin is required for entering the cell and for activating the read out based on the single chain luciferase fusion polypeptide of the present invention. Accordingly, such a fully biologically active neurotoxin is to be applied if a host cell is to be contacted with the sample to be analyzed by the method of the invention. In another aspect, the sample to be applied for the method of the invention comprises neurotoxin polypeptides or fragments thereof which merely exhibit neurotoxin protease activity. Such neurotoxin polypeptides or fragments are, in an aspect, muteins of neurotoxin polypeptides comprising or consisting essentially of a proteolytically active light chain. It is to be understood that samples comprising neurotoxin polypeptides or fragments thereof which merely exhibit neurotoxin protease activity shall be used if the sample is to be contacted to a cell free system as specified elsewhere herein in detail.

The neurotoxin polypeptide in a sample can be determined quantitatively or qualitatively. For a qualitative determination, in an aspect of the invention, the presence or absence of a neurotoxin polypeptide is determined. For a quantitative detection, in an aspect, the amount of the neurotoxin polypeptide is determined. In an aspect, the quantitative determination encompasses a determination of the absolute amount or a relative amount, i.e. an amount which is normalized such that the amount found in different samples can be compared with each other. In an aspect, this can be achieved by comparison of a measured luciferase activity for a test sample to a calibration curve which is to be established by subjecting calibration samples having predetermined amounts of the neurotoxin polypeptide to the method of the present invention.

It will be understood that in an aspect, the neurotoxin cleavage site comprised in the single chain luciferase fusion polypeptide is recognized by the proteolytically active neurotoxin polypeptide to be determined in the sample. In yet an aspect, said neurotoxin polypeptide is selected from the group consisting of: BoNT/A, BoNT/B, BoNT/C1, BoNT/D, BoNT/E, BoNT/F, BoNT/G or TeNT.

In another aspect of the method of the invention, the activity of more than one neurotoxin polypeptide shall be determined. To this end, a host cell can be applied which comprises a polynucleotide according to the present invention encoding a first single chain luciferase fusion polypeptide comprising: (i) a LuxB subunit of a first luciferase, (ii) a linker comprising a neurotoxin cleavage site for a first neurotoxin polypeptide, and (iii) a LuxA subunit of said first luciferase and another polynucleotide according to the present invention encoding a second single chain luciferase fusion polypeptide comprising: (i) a LuxB subunit of a second luciferase, (ii) a linker comprising a neurotoxin cleavage site for a second neurotoxin polypeptide, and (iii) a LuxA subunit of said second luciferase. It will be understood that the first and the second neurotoxin polypeptides are different and recognize and cleave different cleavage sites. Moreover, in an aspect it will be understood that the first luciferase holoenzyme comprising said first LuxB and LuxA subunits and the second luciferase holoenzyme comprising said second LuxB and LuxA subunits utilize generate different light emissions which can be distinguished from each other, e.g., emission maxima at different wavelengths. In an aspect, said first luciferase holoenzyme and/or said second luciferase holoenzyme can be bound to a fluorescent protein, e.g. green fluorescent protein (GFP), yellow fluorescent protein (YFP), red fluorescent protein (RFP), blue fluorescent protein, and the like. In an aspect, the first subunits are from Vibrio harveyi and the second subunits referred to before are from Vibrio fischeri.

Thus, the method of the present invention allows for an efficient determination of a biologically active neurotoxin polypeptide in a sample and can, therefore, be applied in high throughput screenings or quality control approaches. Thanks to the method of the present invention, neurotoxin polypeptide determination can be automated due to the use of cell culture cells rather than primary neurons. Moreover, the use of stably transfected host cell lines allows for a comparable quality of the readout system, i.e. the host cell to be applied in the method of the invention. Accordingly, the method of the present invention may serve as an alternative or may at least significantly reduce animal testing in the context of neurotoxin polypeptide development or quality control.

Further encompassed by the present invention is the use, in general, of the polynucleotide, the vector, the host cell or the polypeptide of the invention for determining a proteolytically active neurotoxin polypeptide in a sample in vitro.

Finally, the present invention contemplates a kit for determining a proteolytically active neurotoxin polypeptide in a sample comprising the polynucleotide, the vector, the host cell and/or the polypeptide of the present invention and, preferably, a detection agent for measuring the enzymatic conversion of a luciferase substrate and a luciferase substrate.

The term "kit" as used herein refers to a collection of means comprising the polypeptide, the polynucleotide, the vector and/or the host cell of the present invention which are provided in separate or common vials in a ready to use manner for carrying out the method of the present invention. In an aspect, the kit comprises additional means for carrying out the method of the present invention, in an aspect, calibration standard solutions comprising neurotoxin polypeptide and/or means for measuring the luciferase activity such as detection agents for luciferase or substrates converted by luciferase. Furthermore, in an aspect, the kit comprises instructions for carrying out the method of the present invention. These instructions can be provided as a manual or can be in the form of an computer-implementable algorithm on a data storage medium which upon implementation is capable of governing one or more steps of the method of the invention. In an aspect, the kit is to be used for carrying out the method of the invention specified above.

### FIGURES

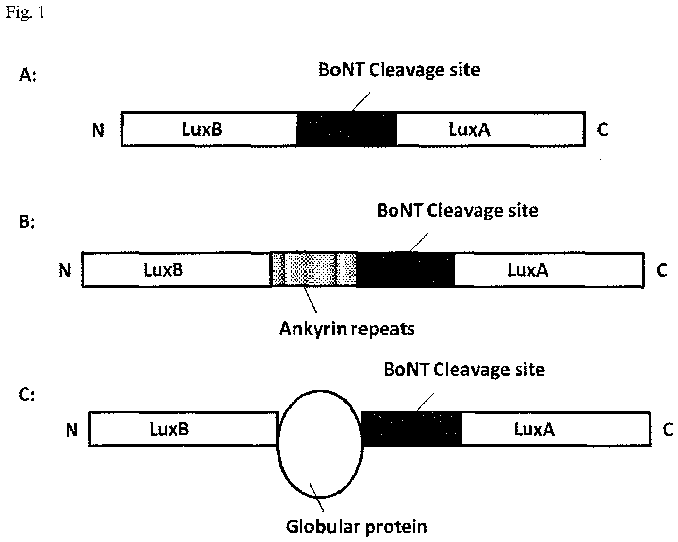

**Figure 1** shows a schematic drawing of the single chain luciferase fusion polypeptide of the in vention, (A) prevention of the self interaction of the subunits by a specific order of the said subunits; (B) prevention of the self interaction by ankyrin repeats; (C) prevention of the self interaction by globular proteins.

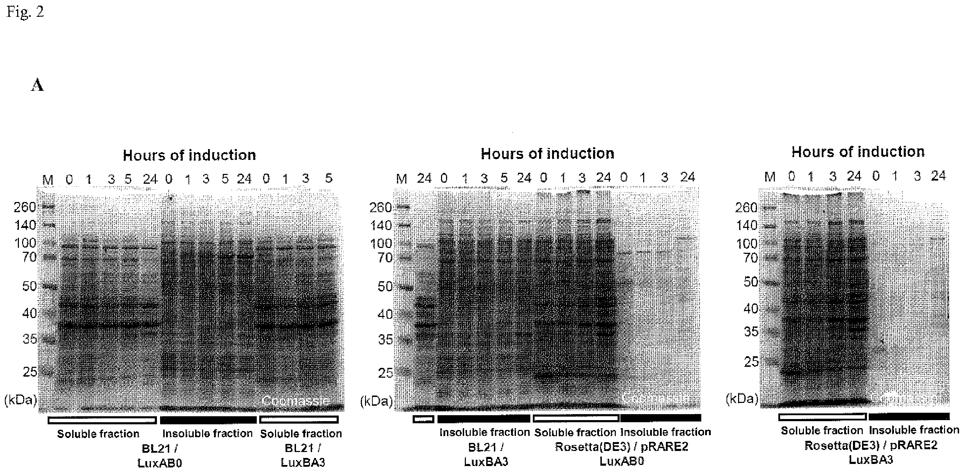

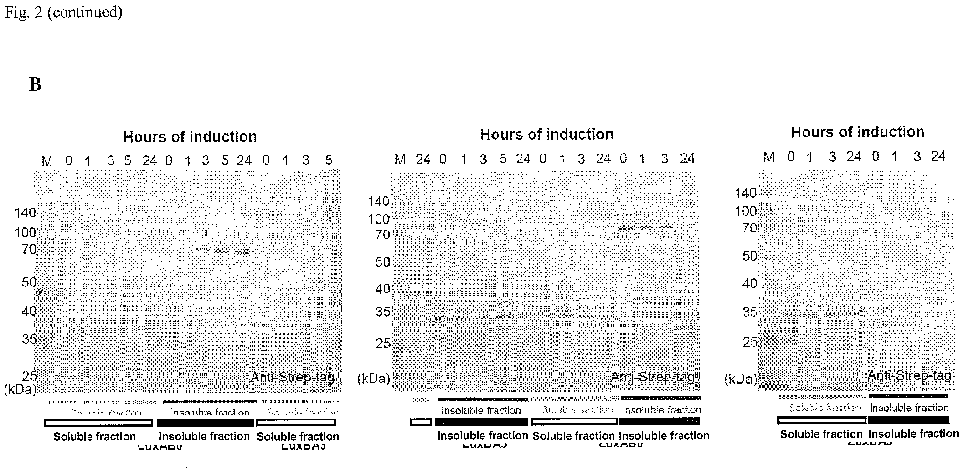

**Figure 2** shows the expression of MRZ_LuxAB0 and MRZ_LuxBA3 in different *E. coli* expression strains at 22°C, whereby soluble and insoluble fractions were separated. Load per lane: 1/6 x OD₆₀₀ unit with 1 x OD₆₀₀ unit defined as 1 ml of a culture with an OD₆₀₀ of 1.0. Selected marker sizes are depicted to the left. **A** shows the 12% SDS-PAGE analysis of the expression of MRZ_LuxAB0 and MRZ_LuxBA3 in the *E. coli* strains BL21 and Rosetta (DE3) / pRARE2. Gels were stained with Coomassie Blue. **B** shows the Western-blot analysis of a 12% gel; detection was performed with anti-Strep-tag antibody, secondary antibody anti mouse-AP, developed with NBT / BCIP.

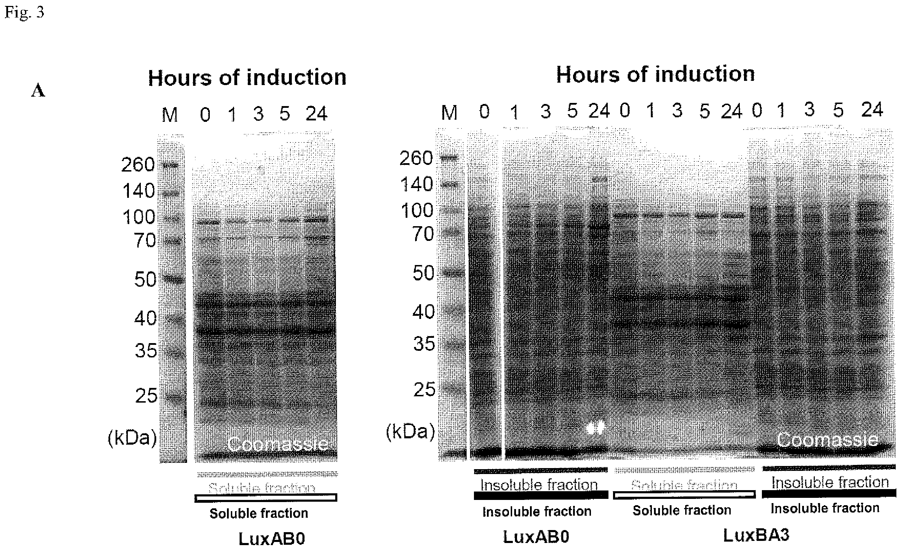

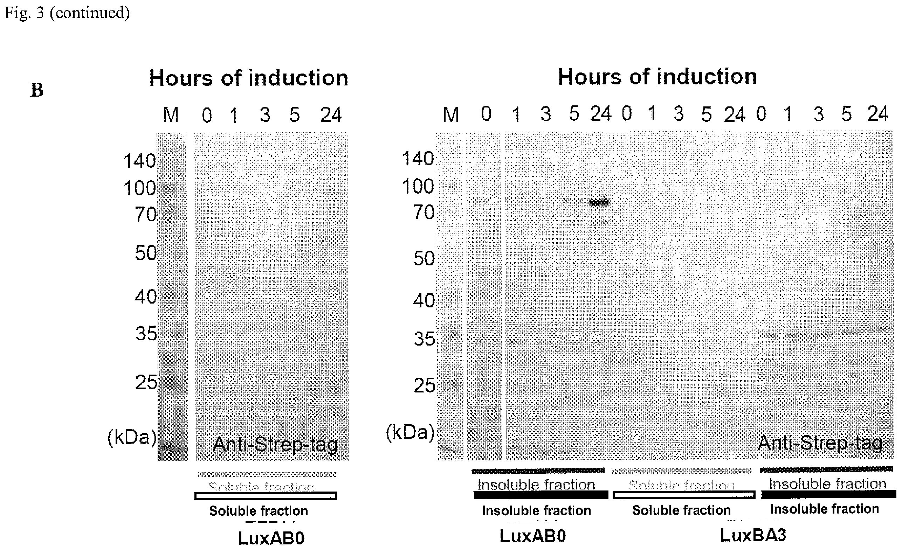

**Figure 3** shows the expression of MRZ_LuxAB0 and MRZ_LuxBA3 in *E. coli* expression strain BL21 at 16°C, whereby soluble and insoluble fractions were separated. Load per lane: 1/6 x OD₆₀₀ unit with 1 x OD₆₀₀ unit defined as 1 ml of a culture with an OD₆₀₀ of 1.0. Selected marker sizes are depicted to the left. **A** shows the 12% SDS-PAGE analysis of the expression of MRZ_LuxAB0 and MRZ_LuxBA3 in the *E. coli* strain BL21. Gels were stained with Coomassie Blue. **B** shows the Western-blot analysis of a 12% gel; detection was performed with anti-Strep-tag antibody, secondary antibody anti mouse-AP, developed with NBT / BCIP.

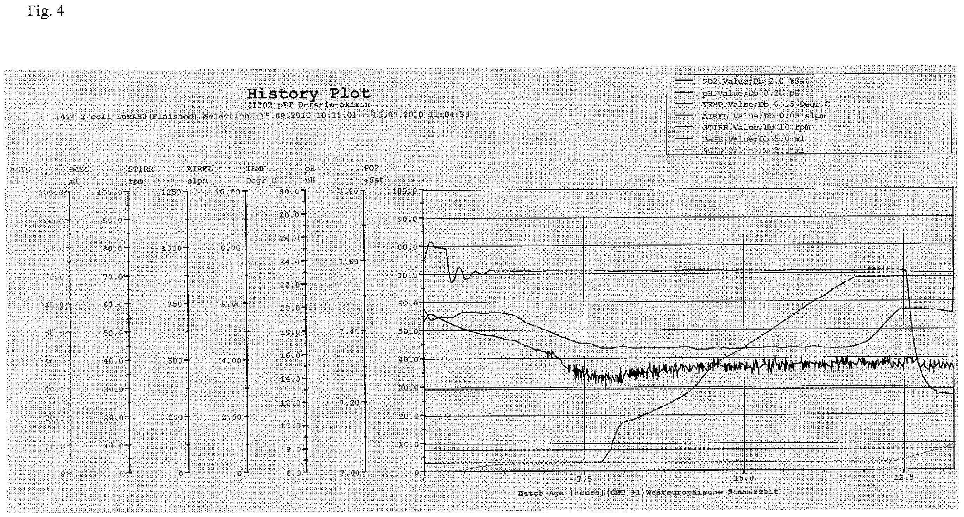

**Figure 4** shows *E. coli* strain BL21 LuxAB0 fed-batch fermentation. One 5 l fermentation was run to generate 62 g (wcw) biomass.

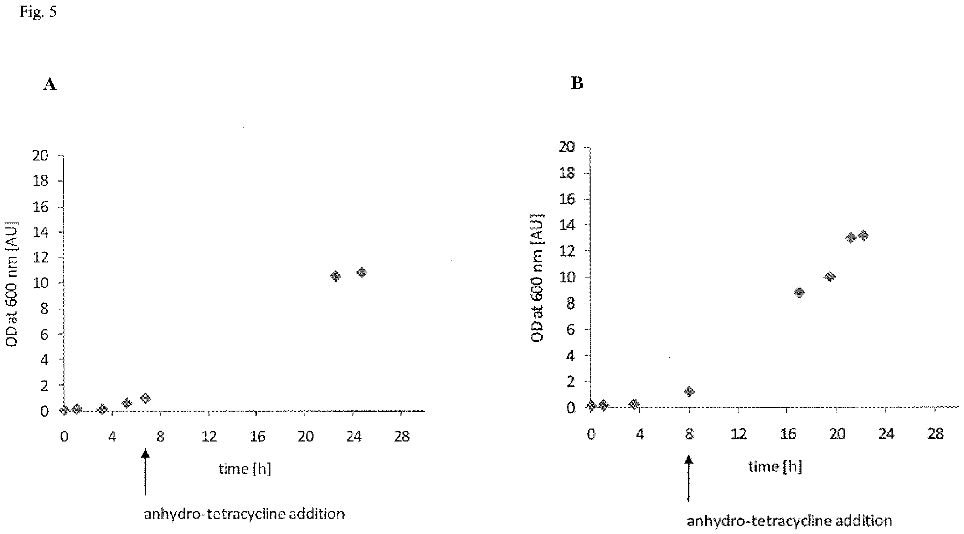

**Figure 5** shows. OD₆₀₀ throughout fed-batch fermentation of **A** *E. coli* strain BL21 LuxAB0 and **B** *E. coli* strain BL21 LuxBA1.

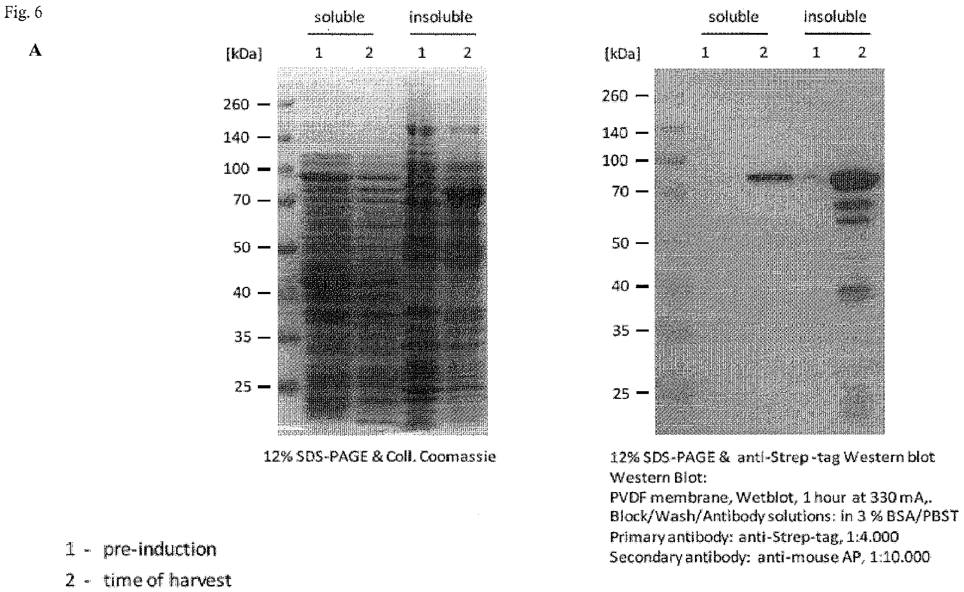

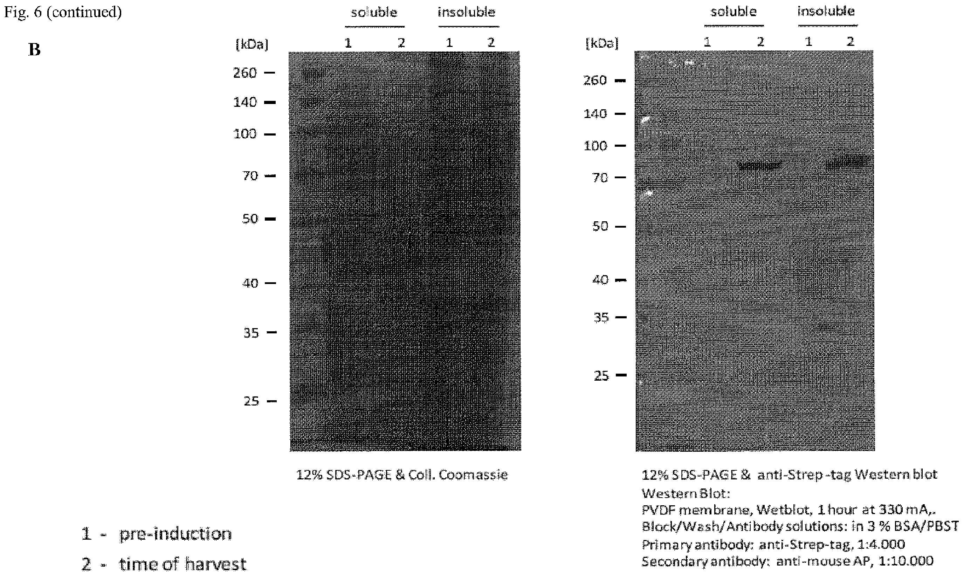

**Figure 6** shows SDS-PAGE and Western blotting of samples from before induction and at time of harvest of **A** *E. coli* strain BL21 LuxAB0 and **B** *E*. *coli* strain BL21 LuxBA1.

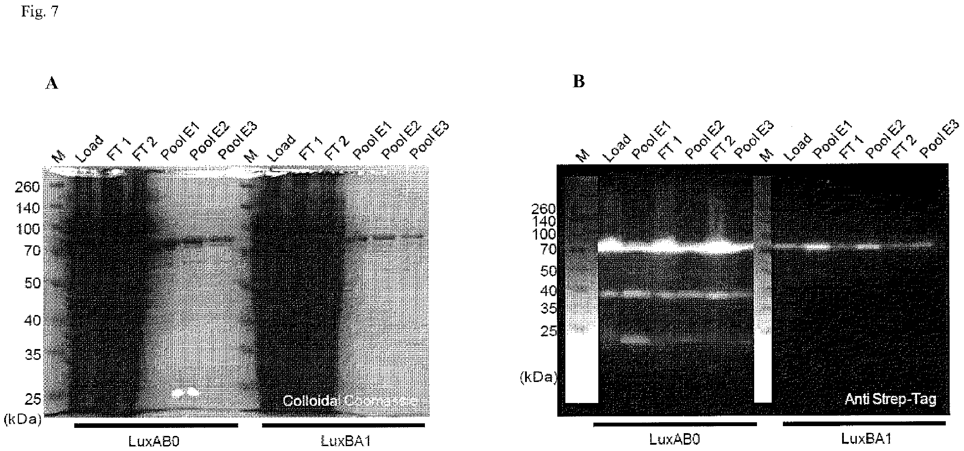

**Figure 7** shows Strep-tag affinity batch-purification of BL21-MRZ-LuxAB0 and BL21-MRZ-LuxBA1. Load per lane is 1.25 µl undiluted sample of fractions Load, FT1, and FT2, and 2.5 µl undiluted sample of Pool E1, Pool E2, and Pool E3. Selected marker sizes are depicted to the left. **A** SDS-PAGE analysis; Gel 12%, stained with Coomassie Blue. **B** Western-blot analysis; Gel 12%, detection with anti-Strep antibody (StrepMAB-Classic, HRP conjugate, IBA, Cat. No. 2-1509-001, dilution 1:10000 in PBS-Tween containing 2% BSA).

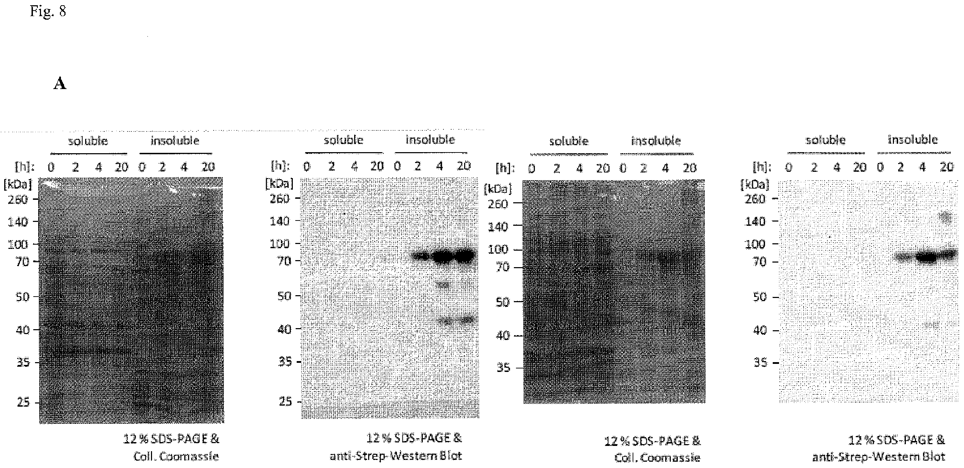

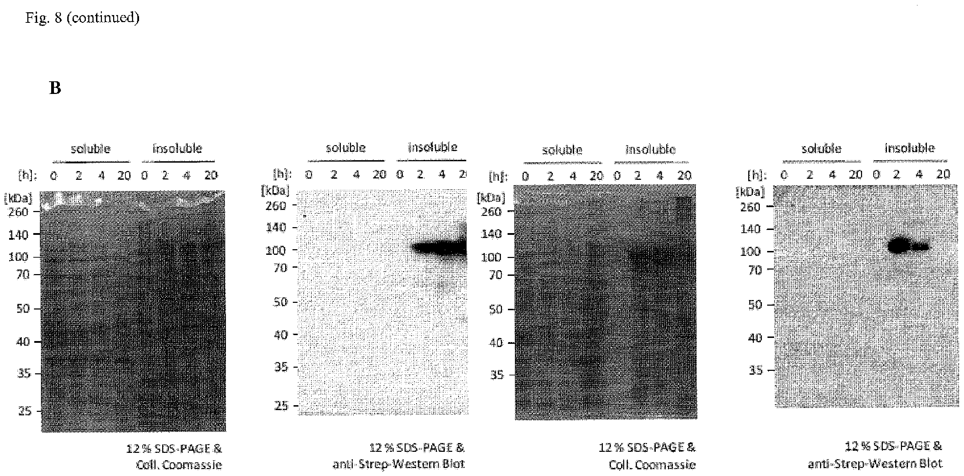

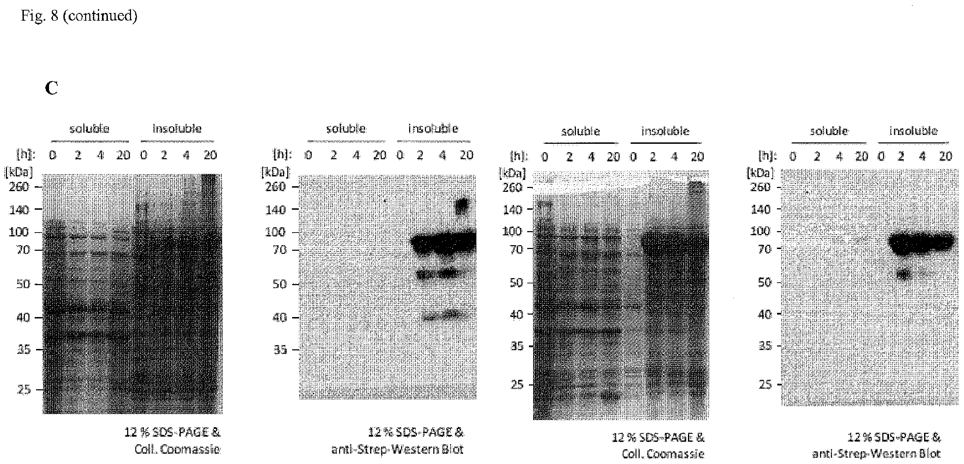

**Figure 8** shows the analysis of expression in small scale *E. coli* shake flask cultures. Target vectors were expressed in the host strains BL21(DE3) and BL21(DE3) Rosetta, and cultured at 37°C. Samples for analysis were drawn just before induction as well as 2, 4, and 20 hours after induction of target expression. Load per lane: 0.25 x OD₆₀₀ units with 1 x OD₆₀₀ unit defined as 1 ml of a culture with an OD₆₀₀ of 1.0. Left side shows BL21(DE3) and right side shows BL21(DE3) Rosetta. **A** shows LuxBA3-Strep (84.9 kDa), **B** GST-LuxBA3-Strep (111.2 kDa), and C 9xHis-LuxBA3-Strep (86.8 kDa).

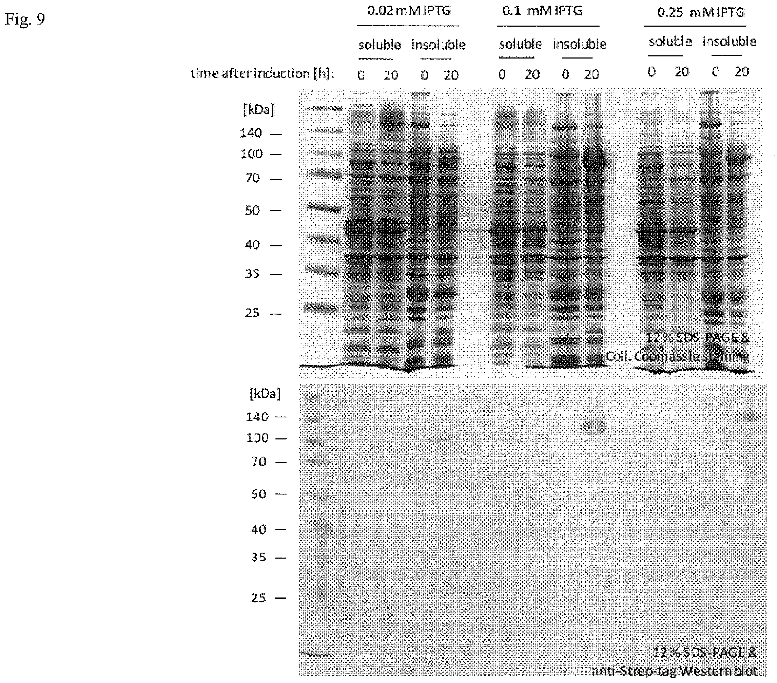

**Figure 9** shows the analysis of 9xHis-Xa-LuxBA3-Strep protein expression. 1 1 shake flask culture grown at 25 °C (before and after induction). 12 % SDS-PAGE followed by Coll. Coomassie-staining and anti-Strep-tag Western blotting. Load per lane: 0.25 x OD₆₀₀ units with 1 x OD₆₀₀ unit defined as 1 ml of a culture with an OD₆₀₀ of 1.0. Marker: Fermentas PageRuler™ Prestained Protein Ladder.

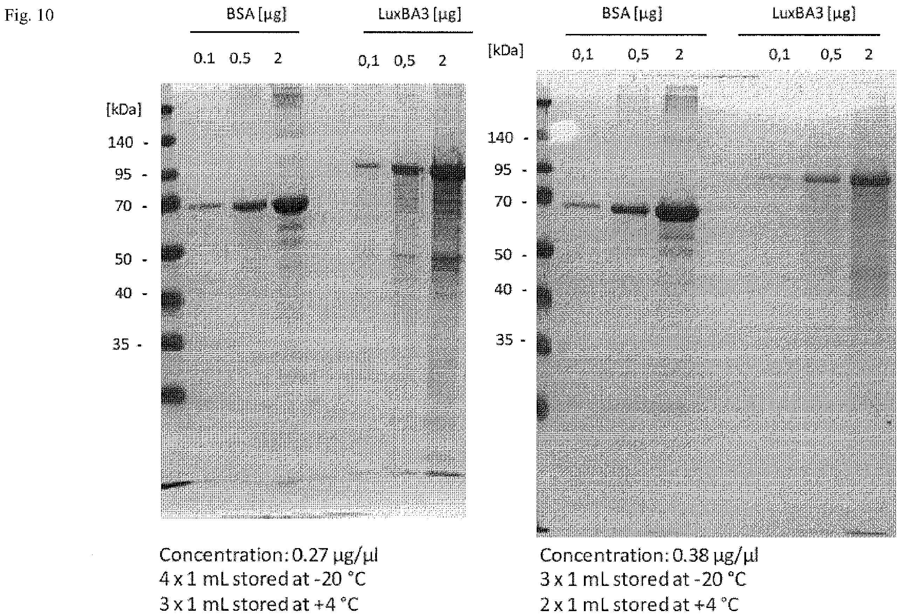

**Figure 10** shows the analysis of final LuxBA3 protein samples. 12% SDS-PAGE and Coll. Coomassie staining. The two different gels refer to two independent FPLC runs (left panel: 1.89 mg total; right panel: 1.90 mg total) but from the same starting material.

### EXAMPLES

The invention will now be illustrated by Examples which shall, however, not be construed as limiting the scope of the invention.

### Example 1: Expression, fermentation and purification of different Luciferase constructs in Escherichia coli

### Expression of MRZ_LuxAB0 and MRZ_LuxBA3 in E. coli

The constructs MRZ_LuxAB0 (SEQ ID NO: 9) and MRZ_LuxBA3 (SEQ ID NO: 10) are pASK-IBA3-plus vector constructs, encoding for two different Luciferase-targets both carrying a C-terminal Strep-tag II. The expected molecular weight of Luciferase AB0 carrying a C-terminal Strep-tag-II is 78.6 KDa, with an estimated pI of 5.22. The expected molecular weight of Luciferase BA3 carrying a C-terminal Strep-tag-II is 84.9 KDa, with an estimated pI of 5.20.

### Expression of MRZ_LuxAB0 and MRZ_LuxBA3 at 22°C

For analyzing the expression MRZ_LuxAB0 and MRZ_LuxBA3 (both Amp^{R}) were transformed into the *E. coli* strains BL21 and Rosetta (DE3) / pRARE2 (Cam^{R}). Cells were grown at 37°C in LB medium supplemented with 200 µg/ml ampicillin (and 30 µg/ml chloramphenicol for the respective strains) until an OD₆₀₀ of 0.4 (BL21) or an OD₆₀₀ of 0.2 (Rosetta (DE3) / pRARE2) was reached. Each culture was shifted to 22°C and grown until an OD₆₀₀ of 0.65 (BL21) or an OD₆₀₀ of 0.3 (Rosetta (DE3) / pRARE2) was reached. Cultures were induced with 0.2 µg/ml anhydrotetracycline and grown for another 24 hours at 22°C. After 0, 1, 3, 5, and 24 hours of induction samples were taken and treated with Bug buster HT solution (Novagen) to break the cells and separate soluble and insoluble protein fractions. Samples were analyzed via SDS-PAGE analysis on 12% Gels (and via Western-blot with anti-Strep-tag antibody), as seen in Figures 2A and 2B.

### Expression of MRZ_LuxAB0 and MRZ_LuxBA3 at 16°C

For analyzing the expression MRZ_LuxAB0 and MRZ_LuxBA3 (both Amp^{R}) were transformed into *E. coli* BL21. Cells were grown at 25°C in LB medium supplemented with 200 µg/ml ampicillin until an OD₆₀₀ of 0.15 was reached. Each culture was shifted to 16°C and grown until an OD₆₀₀ of 0.2 (MRZ_LuxAB0) or an OD₆₀₀ of 0.35 (MRZ_LuxBA3) was reached. Cultures were induced with 0.2 µg/ml anhydrotetracycline and grown for another 24 hours at 16°C. After 0, 1, 3, 5 and 24 hours of induction samples were taken and treated with Bug buster HT solution (Novagen) to break the cells and separate soluble and insoluble protein fractions. Samples were analysed via SDS-PAGE analysis on 12% Gels (and via Western-blot with anti- Strep-tag antibody), as seen in Figures 3A and 3B.

To clearly relate bands in the Coomassie stained gels shown in Figures 2A and 3A to the Luciferase AB0 and BA3 target and to estimate the amount of soluble compared to insoluble target, western blot analysis with anti- Strep-Tag antibody was performed. The western-blot analysis revealed the presence of a protein running between the 75 and the 100 kDa molecular weight marker band (corresponding with the expected molecular weight of 78.6 kDa of the translated Luciferase AB0 protein) in the insoluble protein fraction of the BL21 / LuxAB0 expression, as shown in Figures 2B and 3B. The LuxAB0-construct showed the best soluble expression in BL21 after 24 hours of induction at 16°C. In both Figures no specific western-blot signal was detected by the anti-Strep-tag antibody in the LuxBA3 expression strains. In Figure 3B, however, a very weak signal can be seen after 24 hours of expression in BL21 in the soluble protein fraction.

### High cell density batch-fermentation of the E. coli strains BL21 LuxAB0 and BL21 LuxBA1

*E. coli* strains BL21 LuxAB0 and BL21 LuxBA1 were separately grown in batch fermentation mode. A single colony of each strain was picked from an LB plate, inoculated in 2 x 100 ml LB medium, grown at 25°C, 175 rpm for 16 hours. These preculture were used to inoculate 4.5 1 fermentation medium. Ampicillin was added in all cultures to 100 µg/ml. Growth was recorded throughout the fermentation (Figures 4, 5A, and 5B). Fermenter settings are summarized in Table 1.

**Table 1 summarizes fermenter settings.**

| Fermenter settings summary: | |
|---|---|
| Preculture volume | 125 ml |
| Initial fermentation volume | 4.51 |
| pH | 7.4 (adjusted using ammonia/phosphoric acid) |
| pO₂ | 20 % (stir → airflow cascade) |
| Temperatur | 23 °C whole cultivation period |
| Antifoam reagent | Antifoam A (Sigma) at 1 ml/l |
| Antibiotic | Ampicillin at 100 µg/ml |
| Inducer | Anhydro-tetracycline at 1 mg/l |

The fermentation medium was made as follows (per liter): For YTG base, to 900 ml of H₂O add 12 g bacto-tryptone, 24 g bacto-yeast extract, and 4 mL glycerol. In a separate flask dissolve in 90 mL H₂O 2.31 g KH₂PO₄ monobasic, 12.54 g K₂HPO₄ dibasic, and adjust volume to 100 mL with H₂O. Both solutions were autoclaved separately and mixed only after cooling down to below 60°C.

The fermenter cultures were inoculated to a starting OD₆₀₀ of about 0.1 at 23°C which was kept throughout the whole fermentation process. The culture of *E. coli* strain BL21 LuxAB0 had reached an OD₆₀₀ of 0.96 after 7 hours, wherein the culture of *E. coli* strain BL21 LuxBA1 had reached an OD₆₀₀ of 1.2 after 8 hours. Then the inducer anhydro-tetracycline was added (1.0 mg/l final concentration). The cultures were harvested after an additional 18 hours (LuxABO) or 15 hours (LuxBA1) by centrifugation at 8.000 g for 20 min at 4 °C. The supernatant was discarded, the cell pellets snap frozen in liquid nitrogen and then stored at -80 °C until further use. The final OD₆₀₀ were 10.8 (LuxABO) and 13.5 (LuxBA1) with a culture volume of about 5 l. The biomass yield were 62 g (wcw, LuxAB0) and 70 g (wcw, LuxBA1).

During the fermentation, two samples were drawn (just before anhydro-tetracycline addition and at the time of harvest), the cells pelleted by centrifugation, and then also stored at -80 °C until further use. These two samples were processed for analysis using Bugbuster (Novagen) to separate soluble from insoluble material. Comparable amounts were analyzed by SDS-PAGE and subsequent Colloidal Coomassie staining and Western blotting, respectively (Figures 6A and 6B; load per lane: 0.25 x OD₆₀₀ units for Coomassie staining and 0.5 x OD₆₀₀ units for anti-Strep-tag Western blotting, with 1 x OD₆₀₀ unit defined as 1 ml of a culture with an OD₆₀₀ of 1.0.)

### Strep-tag affinity batch-purification of LuxAB0 and LuxBA1

62 g (wcw, LuxAB0) fermenter biomass or 70 g (wcw, LuxBA1) fermenter biomass were resuspended in 150 ml resuspension buffer (100 mM Tris/HCl pH 8.0, 150 mM NaCl, and 1 mM EDTA). The cells were broken by passing them two times through a microfluidizer. Unbroken cells were removed by centrifugation at 4°C, 10000 x g for 30 minutes (pellet was discarded, supernatant = Load). 1 ml (bed volume) Strep-Tactin Superflow matrix from IBA was added to the crude extract (supernatant, Load) and binding was performed for 30 minutes with gentle shaking at 4°C. The suspension was centrifuged at 4°C, 2000 x g for 10 minutes. The matrix was transferred to a gravity column, the flowthrough was collected (FT1). The column was washed one time with 5 ml of resuspension buffer (wash was added to the FT1) followed by six elution steps with 500 µl resuspension buffer containing 2.5 mM D-desthiobiotin (E1 - E6, first Elution). The flow-trough (FT1) was loaded on the column again. The flow-through of this step was collected again (FT2). The column was washed one time with 5 ml of resuspension buffer (wash was added to the FT2). A second elution was performed consisting of six elution steps with 500 µl resuspension buffer containing 2.5 mM D-desthiobiotin (E1 - E6, second Elution). The second flow-trough (FT2) was loaded on the column again. A third elution was performed consisting of six elution steps with 500 µl resuspension buffer containing 2.5 mM D-desthiobiotin (E1 - E6, third Elution). The following elution fractions were pooled: Pool E1 (E1-E6 from elution 1), Pool E2 (E1-E6 from elution 2), and Pool E3 (E1-E6 from elution 3). Samples were analysed via 12% SDS-PAGE analysis and western blot using Strep-tag antibody (StrepMAB-Classic, HRP conjugate, IBA, Cat. No. 2-1509-001), as shown in Figure 7.

Protein concentrations of the elution fractions were determined using Bradford analysis. Each elution fraction pool was split in two halves (with 1,5 ml each respectively) and stored at 4°C. The total protein yield of the elution fractions were approximately 6 mg for the LuxAB0 construct (purified out of 62g wcw fermenter biomass) and approximately 3 mg for the LuxBA1 construct (purified out of 70g wcw fermenter biomass), as shown in Table 2.

**Table 2 shows total protein yield for LuxAB0 and LuxBA1 constucts.**

| Eluation pool | Concentration [µg/µl] | Volume [ml] | Total protein yield [mg] |
|---|---|---|---|
| LuxAB0 E1 | 1.00 | 3.00 | 3.00 |
| LuxAB0 E2 | 0.53 | 3.00 | 1.59 |
| LuxAB0 E3 | 0.45 | 3.00 | 1.35 |
| LuxBA1 E1 | 0.40 | 3.00 | 1.20 |
| LuxBA1 E2 | 0.36 | 3.00 | 1.08 |
| LuxBA1 E3 | 0.25 | 3.00 | 0.75 |

### Example 2: Cloning of different LuxBA3-Strep-tag expression vectors and expression in E. coli

### Cloning of expression vectors

Using the template DNA (SEQ ID NO: 10), the target sequence was amplified and subcloned into pET-based expression vectors. The resulting target vectors were named accordingly (Table 3). *E. coli* transformants were screened, and plasmid DNA from several candidates was isolated and sequenced. Their target sequences were verified by DNA sequencing.

**Table 3 shows nomenclature and SEQ ID NOs. of generated expression vectors.**

| Expression vector | Protein features (N- to C-terminal) | Resis tance | Protein sequence | DNA vector sequence |
|---|---|---|---|---|
| pTZ_E02_LuxBA3 | LuxBA3-Strep | Kan | SEQ ID NO: 11 | |
| pTZ_E30_LuxBA3 | GST-LuxBA3-Strep | Amp | SEQ ID NO: 12 | |
| pTZ_E47_LuxBA3 | 9xHis-LuxBA3-Strep | Kan | SEQ ID NO: 13 | SEQ ID NO: 14 |

| | | | | |
|---|---|---|---|---|
| Abreviations: His, Histidin; Strep, Streptavidin-tag; Kan, Kanamycin; Amp, Ampicillin. | | | | |

### Expression in small scale E. coli shake flask cultures

Each of the three constructs listed in Table 3 were expressed in two different host strains. Samples for analysis were drawn at 4 timepoints (just before IPTG addition as well as 2, 4, and 20 hours post-induction). A single colony was picked from an LB plate, inoculated in 5 ml LB medium (incl. the appropriate antibiotics), and grown overnight at 37°C, 175 rpm. From these, fresh 30 ml LB cultures were inoculated to a starting OD₆₀₀ of 0.1. When the cultures reached an OD₆₀₀ of about 0.4, each culture was kept at 37 °C and target expression was induced in all cultures by the addition of IPTG (0.5 mM). All samples were processed in the same manner using Bugbuster (Novagen) to separate soluble from insoluble material. Comparable amounts were analyzed by SDS-PAGE and subsequent Colloidal Coomassie staining and anti-Strep Western blotting, respectively (Figures 8A-C).

### Example 3: Expression of LuxBA3 construct and purification of LuxBA3 protein

### Expression of the LuxBA3 construct

The construct pTZ_E47_LuxBA3 (9xHis-Xa-LuxBA3-Strep protein; see Table 3) was expressed in *E. coli* strain BL21(DE3). A single colony was picked from an LB plate, inoculated in 5 ml LB medium (incl. Kanamycin, 25 µg/ml), and grown overnight at 25 °C, 175 rpm. On the next morning, 100 ml LB shake flask cultures were inoculated to a starting OD₆₀₀ of 0.1. When the cultures reached an OD₆₀₀ of about 0.6, target expression was induced in all cultures by the addition of IPTG (0.02, 0.10, and 0.25 mM IPTG, respectively). Samples for analysis were drawn just before IPTG addition and 20 hours post-induction. These expression conditions were used to generate additional biomass (3 x 11).

All samples were processed in the same manner using Bugbuster (Novagen) to separate soluble from insoluble material. Comparable amounts were analyzed by SDS-PAGE and subsequent Colloidal Coomassie staining and Western blotting, respectively (Figure 9). Beyond the sampling over the time course, the cultures were harvested 20 hours post-induction by centrifugation at 5.000 g for 15 min. Cell pellets were stored at -20°C.

### Purification of the target protein 9xHis-Xa-LuxBA3-Strep (87 kDa) from the insoluble fraction under denaturing conditions - refolding on column

For the purification from the insoluble fraction, all three cell pellets (see above) were combined and processed at once. After binding of the denatured target protein on a NiNTA chromatography column, the target was refolded on column and then eluted. Final yield of purification was 3.8 mg with an estimated purity of 90 %..

The following protocol was used to solubilize the insoluble protein fraction: The biomass was resuspended in PBS pH 7.4 including protease inhibitors. Mechanical cell lysis was performed by passing the resuspended biomass through a microfluidizer. Cell were centrifuged to separate insoluble and soluble fraction. After centrifugation in PBS, the pellet (insoluble fraction) was resuspended, urea was added to 8 M final concentration, and the mixture was incubated for one hour at room temperature with stirring. After a centrifugation at room temperature an urea-insoluble (pellet) and an urea-soluble fraction (supernatant) were obtained. The urea-soluble fraction (supernatant) was loaded onto Nickel-chelating resin (FPLC) using a loading buffer containing 8 M urea). In the next step a linear gradient starting from 8 M to 0 M urea follows over 2 hours. After washing with PBS (no urea from this step onwards) a second washing with PBS and 20 mM Imidazole followed. The proteins are elutated by a linear gradient from 20 to 500 mM Imidazole (in PBS). The final samples were analyzed by SDS-PAGE and Coll. Coomassie staining (Figure 10). Purified target protein was stored in PBS pH 7.4, residual imidazole. Samples were aliquoted. About one half each was stored at +4 °C and the other half frozen and stored at -20 °C.

Further, analysis of the 9xHis-Xa-LuxBA3-Strep protein revealed that the protein is cleavable by BoNT/A activity but that this cleavage did not result in any luciferase activity as desired.

## Claims

1. A polynucleotide encoding a single chain luciferase fusion polypeptide comprising from the N-terminus to the C-terminus: (i) a LuxB subunit, (ii) a linker comprising a neurotoxin cleavage site, and (iii) a LuxA subunit, wherein said luciferase subunit LuxA and LuxB are from a bacterial luciferase.

2. The polynucleotide of claim 1, wherein said luciferase subunit LuxA and LuxB are from Vibrio fischeri or Vibrio harveyi.

3. The polynucleotide of claim 1 or 2, wherein said neurotoxin cleavage site is selected from the group consisting of: a neurotoxin cleavage site from SNAP-25, a neurotoxin cleavage site from VAMP, a neurotoxin cleavage site from syntaxin, and the autocatalytic cleavage site from the neurotoxin polypeptide.

4. A vector comprising the polynucleotide of any one of claims 1 to 3.

5. The vector of claim 4, wherein said vector is an expression vector.

6. A host cell comprising the polynucleotide of any one of claims 1 to 3 or the vector of claim 4 or 5.

7. The host cell of claim 6, wherein said host cell is a cell capable of translocating a neurotoxin polypeptide into its cytoplasm.

8. The host cell of claim 7, wherein said host cell is selected from the group consisting of: neuroblastoma cell lines and primary neurons.

9. A polypeptide encoded by the polynucleotide of any one of claims 1 to 3.

10. A method for determining a proteolytically active neurotoxin polypeptide in a sample comprising:
a) contacting the host cell of any one of claims 6 to 8 or the polypeptide of claim 9 with a sample suspected to comprise said proteolytically active neurotoxin polypeptide under conditions which allow for proteolytic cleavage of the single chain luciferase fusion protein into separate LuxB and LuxA subunits;
b) allowing the said LuxB and LuxA subunit to form a biologically active luciferase; and
c) determining the said luciferase.

11. The method of claim 10, wherein said luciferase is determined by measuring the enzymatic conversion of a luciferase substrate.

12. The method of claim 10 or 11, wherein the neurotoxin cleavage site comprised in the single chain luciferase fusion polypeptide is recognized by the proteolytically active neurotoxin polypeptide to be determined in the sample.

13. The method of any one of claims 10 to 12, wherein said neurotoxin polypeptide is selected from the group consisting of: BoNT/A, BoNT/B, BoNT/C1, BoNT/D, BoNT/E, BoNT/F, BoNT/G or TeNT.

14. Use of the polynucleotide of any one of claims 1 to 3, the vector of claim 4 or 5, the host cell of any one of claims 6 to 8 or the polypeptide of claim 9 for determining a proteolytically active neurotoxin polypeptide in a sample in vitro.

15. A kit for determining a proteolytically active neurotoxin polypeptide in a sample comprising the polynucleotide of any one of claims 1 to 3, the vector of claim 4 or 5, the host cell of any one of claims 6 to 8 and/or the polypeptide of claim 9 and, preferably, a detection agent for measuring the enzymatic conversion of a luciferase substrate and a luciferase substrate.

## Patentansprüche

1. Polynukleotid, welches ein einkettiges Luciferase-Fusionspolypeptid kodiert, umfassend vom N-Terminus zum C-Terminus: (i) eine LuxB-Untereinheit, (ii) einen Linker, umfassend eine Neurotoxin-Spaltstelle, und (iii) eine LuxA-Untereinheit, wobei die Luciferase-Untereinheiten LuxA und LuxB aus bakterieller Luciferase sind.

2. Polynukleotid nach Anspruch 1, wobei die Luciferase-Untereinheiten LuxA und LuxB aus Vibrio fischeri bzw. Vibrio harveyi gewonnen wurden.

3. Polynukleotid nach Anspruch 1 oder 2, wobei die Neurotoxin-Spaltstelle ausgewählt ist aus der Gruppe bestehend aus: einer Neurotoxin-Spaltstelle aus SNAP-25, einer Neurotoxin-Spaltstelle aus VAMP, einer Neurotoxin-Spaltstelle aus Syntaxin und der autokatalytischen Spaltstelle aus dem Neurotoxin-Polypeptid.

4. Vektor, umfassend das Polynukleotid nach einem der Ansprüche 1 bis 3.

5. Vektor nach Anspruch 4, wobei es sich bei dem Vektor um einen Expressionsvektor handelt.

6. Wirtszelle, umfassend das Polynukleotid nach einem der Ansprüche 1 bis 3 oder den Vektor nach Anspruch 4 oder 5.

7. Wirtszelle nach Anspruch 6, wobei es sich bei der Wirtszelle um eine Zelle handelt, die ein Neurotoxin-Polypeptid in ihr Zytoplasma translozieren kann.

8. Wirtszelle nach Anspruch 7, wobei die Wirtszelle ausgewählt ist aus der Gruppe bestehend aus: Neuroblastom-Zelllinien und primären Neuronen.

9. Polypeptid kodiert durch das Polynukleotid nach einem der Ansprüche 1 bis 3.

10. Verfahren zur Bestimmung eines proteolytisch aktiven Neurotoxin-Polypeptids in einer Probe, umfassend:
a) Inkontaktbringen der Wirtszelle nach einem der Ansprüche 6 bis 8 oder des Polypeptids nach Anspruch 9 mit einer Probe, von der vermutet wird, dass sie das proteolytisch aktive Neurotoxin-Polypeptid unter Bedingungen aufweist, welche proteolytische Spaltung des einkettigen Luciferase-Fusionsproteins in getrennte LuxB- und LuxA-Untereinheiten erlauben;
b) Ermöglichen, dass die LuxB- und LuxA-Untereinheiten eine biologisch aktive Luciferase bilden und
c) Bestimmen der Luciferase.

11. Verfahren nach Anspruch 10, wobei die Luciferase bestimmt wird durch Messen der enzymatischen Umwandlung eines Luciferasesubstrats.

12. Verfahren nach Anspruch 10 oder 11, wobei die in dem einkettigen Luciferase-Fusionspolypeptid enthaltene Neurotoxin-Spaltstelle durch das in der Probe zu bestimmende proteolytisch aktive Neurotoxin-Polypeptid erkannt wird.

13. Verfahren nach einem der Ansprüche 10 bis 12, wobei das Neurotoxin-Polypeptid ausgewählt ist aus der Gruppe bestehend aus: BoNT/A, BoNT/B, BoNT/C1, BoNT/D, BoNT/E, BoNT/F, BoNT/G bzw. TeNT.

14. Anwendung des Polynukleotids nach einem der Ansprüche 1 bis 3, des Vektors nach Anspruch 4 oder 5, der Wirtszelle nach einem der Ansprüche 6 bis 8 oder des Polypeptids nach Anspruch 9 zur Bestimmung eines proteolytisch aktiven Neurotoxin-Polypeptids in einer Probe in vitro.

15. Kit zur Bestimmung eines proteolytisch aktiven Neurotoxin-Polypeptids in einer Probe, umfassend das Polynukleotid nach einem der Ansprüche 1 bis 3, den Vektor nach Anspruch 4 oder 5, die Wirtszelle nach einem der Ansprüche 6 bis 8 und/oder das Polypeptid nach Anspruch 9 und vorzugsweise ein Nachweismittel zum Messen der enzymatischen Umwandlung eines LuciferaseSubstrats und ein Luciferase-Substrat.

## Revendications

1. Polynucléotide codant pour un polypeptide de fusion d'une luciférase en simple chaîne comprenant, à partir de l'extrémité N-terminale vers la C-terminale : (i) une sous-unité LuxB, (ii) un lieur qui comprend un site de coupure d'une neurotoxine, et (iii) une sous-unité LuxA, dans lequel lesdites sous-unités LuxA et LuxB de luciférase proviennent d'une luciférase bactérienne.

2. Polynucléotide selon la revendication 1, dans lequel lesdites sous-unités LuxA et LuxB proviennent de Vibrio fischeri ou de Vibrio harveyi.

3. Polynucléotide selon les revendications 1 ou 2, dans lequel ledit site de coupure d'une neurotoxine est choisi dans le groupe constitué par : un site de coupure d'une neurotoxine provenant de SNAP-25, un site de coupure d'une neurotoxine provenant de VAMP, un site de coupure d'une neurotoxine provenant de la syntaxine, et le site de coupure auto-catalytique provenant du polypeptide de neurotoxine.

4. Vecteur comprenant le polynucléotide selon l'une quelconque des revendications 1 à 3.

5. Vecteur selon la revendication 4, ledit vecteur étant un vecteur d'expression.

6. Cellule hôte comprenant le polynucléotide, selon l'une quelconque des revendications 1 à 3, ou le vecteur selon les revendications 4 ou 5.

7. Cellule hôte selon la revendication 6, ladite cellule hôte étant une cellule capable d'effectuer une translocation d'un polypeptide de neurotoxine dans son cytoplasme.

8. Cellule hôte selon la revendication 7, ladite cellule hôte étant choisie dans le groupe constitué par : des lignées de cellules de neuroblastome et des neurones primaires.

9. Polypeptide codé par le polynucléotide selon l'une quelconque des revendications 1 à 3.

10. Procédé de détermination d'un polypeptide de neurotoxine actif au plan protéolytique dans un échantillon, comprenant les étapes consistant à :
a) mettre en contact la cellule hôte, selon l'une quelconque des revendications 6 à 8, ou la polypeptide selon la revendication 9, avec un échantillon suspecté de comprendre ledit polypeptide de neurotoxine actif au plan protéolytique dans des conditions qui permettent de réaliser une coupure protéolytique de la protéine de fusion d'une luciférase en simple chaîne en des sous-unités LuxB et LuxA séparées ;
b) permettre auxdites sous-unités LuxB et LuxA de former une luciférase biologiquement active ; et
c) déterminer ladite luciférase.

11. Procédé selon la revendication 10, dans lequel ladite luciférase est déterminée en mesurant la conversion enzymatique d'un substrat de luciférase.

12. Procédé selon les revendications 10 ou 11, dans lequel le site de coupure d'une neurotoxine compris dans le polypeptide de fusion d'une luciférase en simple chaîne est reconnu par le polypeptide de neurotoxine actif au plan protéolytique devant être déterminé dans l'échantillon.

13. Procédé selon l'une quelconque des revendications 10 à 12, dans lequel ledit polypeptide de neurotoxine est choisi dans le groupe constitué par le : BoNT/A, BoNT/B, BoNT/C1, BoNT/D, BoNT/E, BoNT/F, BoNT/G ou TeNT.

14. Utilisation du polynucléotide selon l'une quelconque des revendications 1 à 3, du vecteur selon les revendications 4 ou 5, de la cellule hôte selon l'une quelconque des revendications 6 à 8, ou du polypeptide, selon la revendication 9, pour déterminer un polypeptide de neurotoxine actif au plan protéolytique dans un échantillon in vitro.

15. Trousse de détermination d'un polypeptide de neurotoxine actif au plan protéolytique dans un échantillon comprenant le polynucléotide selon l'une quelconque des revendications 1 à 3, le vecteur selon les revendications 4 ou 5, la cellule hôte selon l'une quelconque des revendications 6 à 8, et/ou le polypeptide selon la revendication 9, et de préférence un agent de détection destiné à mesurer la conversion enzymatique d'un substrat de luciférase et un substrat de luciférase.
